(19) Europäisches Patentamt
European Patent Office
Office européen des brevets

(11) EP 0 767 778 B1

(12) **FASCICULE DE BREVET EUROPEEN**

(45) Date de publication et mention de la délivrance du brevet: **16.12.1998 Bulletin 1998/51**

(21) Numéro de dépôt: **95924384.1**

(22) Date de dépôt: **29.06.1995**

(51) Int Cl.6: **C07C 233/27**, C07C 217/34, A61K 31/165, A61K 31/135, A61K 51/04

(86) Numéro de dépôt international: **PCT/FR95/00870**

(87) Numéro de publication internationale: **WO 96/00717 (11.01.1996 Gazette 1996/03)**

(54) **DERIVES HALOGENES DE 1-PHENOXY-3-N ALKYLAMINOPROPAN-2-OL UTILISABLES COMME MARQUEURS DES RECEPTEURS beta ADRENERGIQUES MYOCARDIQUES ET COMME (beta-BLOQUANT)**

1-PHENOXY-3-N-ALKYLAMINOPROPAN-2-OL HALOGENIERTE DERIVATE ALS MARKER FÜR BETA-ADRENERGE HERZMUSKELREZEPTOREN SOWIE ALS BETABLOCKER

1-PHENOXY-3-N-ALKYLAMINOPROPAN-2-OL HALOGEN DERIVATIVES USEFUL AS MYOCARDIAL beta ADRENERGIC RECEPTOR MARKERS AND AS beta-BLOCKING AGENTS

(84) Etats contractants désignés: **AT BE CH DE DK ES FR GB GR IT LI LU NL SE**

(30) Priorité: **30.06.1994 FR 9408092**

(43) Date de publication de la demande: **16.04.1997 Bulletin 1997/16**

(73) Titulaire: **CIS BIO INTERNATIONAL**
**91400 Saclay (FR)**

(72) Inventeurs:
- **APPARU, Marcel**
  **F-38400 Saint-Martin-d'Hères (FR)**
- **DEMENGE, Pierre**
  **F-38000 Grenoble (FR)**
- **FAGRET, Daniel**
  **F-38000 Grenoble (FR)**
- **GHEZZI, Catherine**
  **F-38000 Grenoble (FR)**
- **MAJID, Samia**
  **F-38041 Saint-Martin-d'Hères (FR)**
- **MATHIEU, Jean-Paul**
  **F-38360 Sassenage (FR)**
- **MAUCLAIRE, Laurent**
  **F-75013 Paris (FR)**
- **PASQUALINI, Roberto**
  **F-92140 Clamart (FR)**
- **VIDAL, Michel**
  **F-38330 Saint-Nazaire-Les-Eymes (FR)**

(74) Mandataire: **Des Termes, Monique et al**
**Société Brevatome**
**25, rue de Ponthieu**
**75008 Paris (FR)**

(56) Documents cités:
- **NUCL. MED. BIOL., vol. 13, no. 5, 1986 pages 551-555, H. KIZUKA ET AL. 'Synthesis and Biodistribution of 125 Iodine...' cité dans la demande**
- **JOURNAL OF NUCLEAR MEDICINE, vol. 21, no. 5, Mai 1980 NEW YORK US, pages 436-442, WILLIAM C. ECKELMAN ET AL. 'radiochemistry and radiopharmaceuticals' cité dans la demande**
- **NAUNYN-SCHMIEDEBERG'S ARCHIVES OF PHARMACOLOGY, vol. 317, no. 4, 1981 pages 277-285, GÜNTER ENGEL ET AL. '(+/-) [125 Iodo]cyanopindolol, a New Ligand for Beta-Adrenoceptors' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 767 778 B1

## Description

La présente invention a pour objet des dérivés halogénés de 1-phénoxy-3-N-alkylaminopropan-2-ol, utilisables comme marqueurs des récepteurs β adrénergiques myocardiques et comme β-bloquant.

Parmi les différents récepteurs myocardiques, les plus intéressants, d'un point de vue physiologique et physiopathologique, sont les récepteurs β adrénergiques. Ce sont les récepteurs les plus étudiés in vitro, et pour lesquels il existe un grand nombre de données expérimentales.

Ces récepteurs adrénergiques du myocarde sont impliqués dans la régulation et le contrôle des activités mécanique et électrique du coeur, ainsi que dans la régulation du métabolisme cellulaire.

Placés dans la membrane cytoplasmique des cellules cardiaques, ces récepteurs sont activés soit par la noradrénaline libérée in situ par les terminaisons nerveuses sympathiques du myocarde, soit par l'adrénaline circulante libérée par les glandes surrénales.

Cette activation des récepteurs membranaires entraîne une réponse physiologique (augmentation de la fréquence cardiaque, augmentation de la contractilité) dont l'amplitude dépendra du nombre de récepteurs disponibles sur la membrane cellulaire.

Des modifications dans le nombre et/ou dans l'affinité des récepteurs β adrénergiques myocardiques ont été décrites dans de nombreuses pathologies cardiaques telles que l'ischémie et l'infarctus du myocarde, les cardiomyopathies hypertrophiques ou dilatées, le coeur transplanté, et lors d'affections telles que le diabète et l'hypo ou l'hyperthyroïdie.

Ces altérations du nombre des récepteurs β adrénergiques cardiaques entraînent une perturbation du contrôle de l'activité cardiaque que les drogues administrées au patient essaient de compenser.

Toutes ces altérations du nombre de récepteurs β adrénergiques myocardiques ont été mises en évidence in vitro sur des homogénats de tissus provenant d'animaux d'expérience ou sur des biopsies myocardiques réalisées chez l'homme. Mais dans ces conditions, les récepteurs ne sont plus dans leur environnement naturel et n'ont donc plus leurs relations avec les autres composants tissulaires. Il n'existe pas de modèle animal pour toutes les pathologies cardiaques humaines et la variabilité des résultats, mise en évidence selon les espèces animales étudiées, incite à la prudence dans l'extrapolation à l'homme. De plus, les conséquences physiologiques de ces modifications dans le nombre des récepteurs adrénergiques ne peuvent être appréciées par cette technique.

Pouvoir apprécier le nombre de ces récepteurs adrénergiques chez l'homme in vivo permettrait donc :

1 - de suivre l'évolution de la densité des récepteurs adrénergiques au cours de la maladie,
2 - d'apprécier le retentissement, sur cette densité des récepteurs, d'une thérapeutique administrée au patient, et
3 - de comparer cette densité des récepteurs avec les conséquences physiologiques du traitement.

P. Merlet et al ont décrit dans "Circulation, vol. 87, n° 4, 1993, pages 1169-1178, l'emploi de la tomographie par émission de positons pour étudier les récepteurs β adrénergiques myocardiques chez l'homme in vivo en utilisant comme marqueur la (2S)-4 (3-t-butylamino-2-hydroxypropoxy)-benzimidazol-2-one (CGP-12177) marqué au carbone 11, qui répond à la formule :

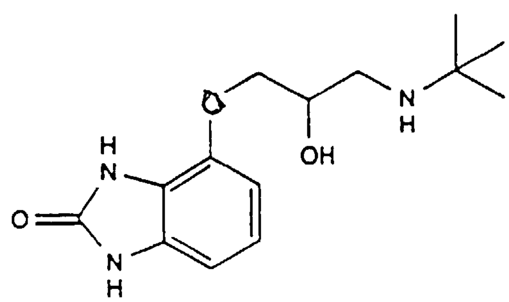

CGP 12177

Avec cette technique, on peut déterminer le nombre de récepteurs β adrénergiques myocardiques, et une diminution de la densité de ces récepteurs a été mise en évidence in vivo chez les patients porteurs d'une cardiomyopathie dilatée primitive.

Cependant, cette technique présente des inconvénients. En effet, l'utilisation de carbone-11 nécessite l'emploi d'un cyclotron pour sa production et, en raison de son émission positonique, l'emploi d'un appareil de détection approprié à l'émission $\beta^+$. De plus, étant donné la demi-vie très courte du carbone-11 (20 minutes) le lieu de production

du traceur marqué au carbone 11 doit être très proche du lieu d'exploration. Aussi, étant donnés les prix d'achat et de fonctionnement de l'appareil de production, de la transformation chimique opérée sur l'isotope et de l'appareil de détection, le coût total d'un examen est très élevé, ce qui en limite considérablement l'emploi.

Des recherches ont donc été entreprises pour remplacer ce marqueur au carbone-11 par des traceurs radioactifs émetteurs γ tels que ceux utilisés classiquement dans les services de médecine nucléaire équipés d'appareils de détection des rayonnements γ routiniers.

Les traceurs susceptibles d'être utilisés peuvent être en particulier des analogues iodés des β bloquants connus pour leur action pharmacologique. Parmi ces β bloquants, des tentatives ont été faites pour marquer à l'iode le practolol, l'alprénolol, le cyanopindolol et le pindolol qui répondent aux formules suivantes :

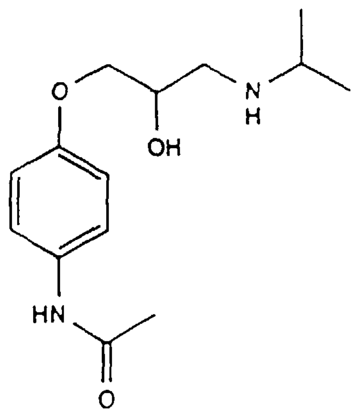

Practolol

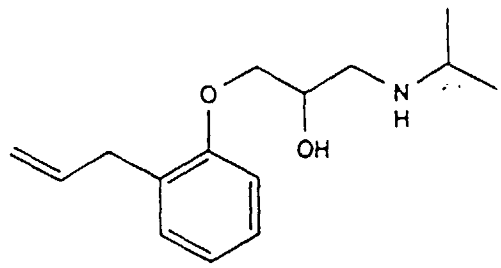

Alprénolol

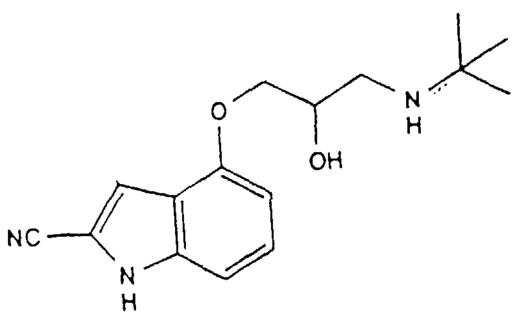

cyanopindolol

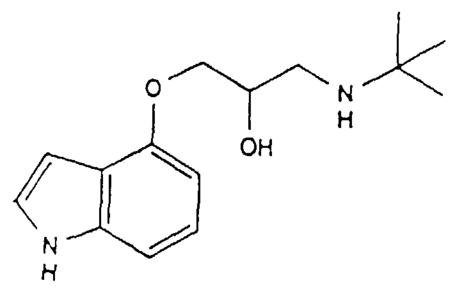

Pindolol

Ainsi, dans J. Nucl. Med., 21, n° 5, 1980, pages 436-442, Eckelman et col ont décrit la synthèse de plusieurs dérivés iodés du practolol et de l'alprénolol dans lesquels l'iode est fixé sur un noyau benzénique (groupe (3-iodo-4-hydroxy) phénétyl) remplaçant le groupe isopropyle du practolol ou de l'alprénolol.

Cependant, des essais effectués avec ces dérivés iodés du practolol ou de l'alprénolol ont montré qu'ils ne présentaient pas une affinité suffisante pour pouvoir être utilisés comme marqueurs des récepteurs β adrénergiques myocardiques.

En revanche, Engel et col. ont décrit dans Naunyn-Schmiedeberg's Arch. Pharmacol., 317, 1981, pages 277-285, que le cyanopindolol marqué à l'iode 125 de formule :

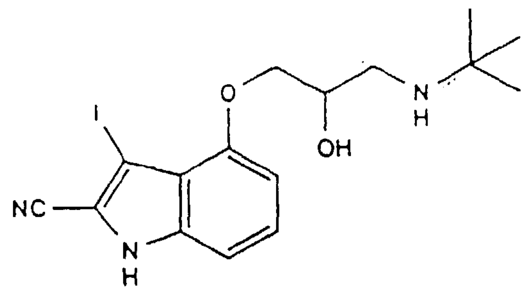

Iodocyanopindolol

pouvait être utilisé in vivo, ce qui n'est pas le cas de l'hydroxybenzylpindolol marqué à l'iode 125 de formule :

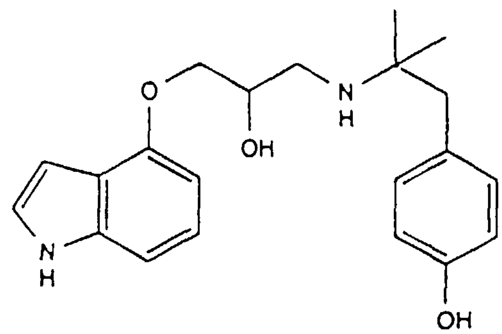

Hydroxybenzylpindolol (HYP)

Dans Nucl. Med. Biol. vol. 13, n° 5, 1986, pages 551-555, Kizuka et col. ont décrit des dérivés iodés du practolol de formules :

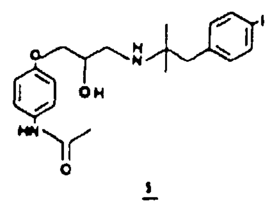

1

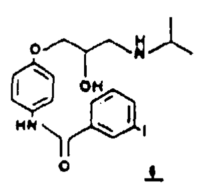

2

dans lesquels l'iode est également fixé sur un cycle benzénique, et ils ont indiqué que de tels ligands ne convenaient pas pour des études in vivo.

Selon la présente invention, on a réalisé d'autres dérivés halogénés du practolol, soit des dérivés de 1-phénoxy-3-N-alkyl aminopropan-2-ol, qui présentent justement de meilleures propriétés que les dérivés connus pour une utilisation comme marqueurs des récepteurs β adrénergiques myocardiques

Selon l'invention, le dérivé halogéné de 1-phénoxy-3-N-alkylaminopropan-2-ol répond à la formule :

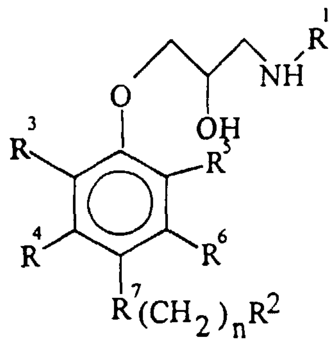

(I)

dans laquelle $R^1$ est un groupe alkyle, un groupe aryle, un groupe aralkyle éventuellement substitué sur sa partie aryle par des groupes alkyle ou alcoxy, ou un groupe $-CH_2-CH_2-Y^1-R^8$, avec $Y^1$ représentant 0, S, NHCO, CONH ou NH-CONH et $R^8$ représentant un groupe alkyle ou aryle éventuellement substitué,

- $R^2$ représente un groupe répondant à l'une des formules suivantes :
- CH=CHX
- C≡CX
- $OCH_2CH=CHX$
- $O(CH_2)_2X$

avec X représentant un atome d'halogène ou un atome d'un isotope radioactif d'un halogène,

- $R^3$, $R^4$, $R^5$ et $R^6$ qui peuvent être identiques ou différents, représentent un atome d'hydrogène, un atome d'halogène, un groupe hydrocarboné saturé ou insaturé, un groupe aryle, OH, CN, $NH_2$, ou un groupe de formule NHR, $NR_2$, COR, CONHR ou COOR avec R représentant un groupe alkyle ou aryle éventuellement substitué,
- $R^7$ représente O ou NHCO, et
- n et égal à 0,1 ou 2.

Dans cette formule, le carbone qui porte le groupe OH peut présenter la configuration soit R, soit S, soit R et S dans des proportions variables.

Dans cette formule, lorsque $R^1$, $R^3$, $R^4$, $R^5$, $R^6$, $R^8$ et R sont des groupes alkyle, ils ont de préférence de 1 à 10 atomes de carbone et ils peuvent être linéaires ou ramifiés.

Pour $R^1$, on utilise de préférence le groupe isopropyle.

Les groupes aryle susceptibles d'être utilisés pour $R^1$, $R^3$, $R^4$, $R^5$, $R^6$, $R^8$ et R sont des groupes aromatiques tels que le groupe phényle.

Lorsque les groupes alkyle ou aryle sont substitués, les substituants peuvent être des atomes d'halogène, des groupes OH, $CONH_2$, $CF_3$, ...

les groupes hydrocarbones saturés ou insaturés utilisés pour $R^3$, $R^4$, $R^5$ et $R^6$ peuvent être des groupes alkyle, alcényle, alcynyle et ils peuvent comporter dans leur chaîne des hétéro-atomes tels que O ou S.

Les groupes aralkyle utilisés pour $R^1$ peuvent être de différents types. A titre d'exemple, on peut citer les groupes benzyle et phénéthyle. Lorsqu'ils sont substitués sur leur partie aryle, les substituants peuvent être des groupes alkyle ou alcoxy, par exemple des groupes méthoxy.

A titre d'exemple de groupe aralkyle substitué, on peut citer le groupe 3,4-diméthyloxy phénéthyle (homovératryle) de formule :

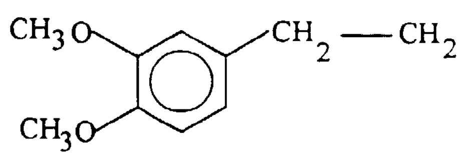

Les atomes d'halogène peuvent être par exemple F, Cl, Br ou I. De préférence, l'atome d'halogène de $R^2$ est I.

Dans cette formule, l'atome d'halogène de $R^2$ peut être en particulier I ou Cl. Lorsqu'on utilise un isotope radioactif de l'halogène, cet isotope peut être $^{123}I$, $^{125}I$, ou $^{131}I$.

Des dérivés halogénés, de formule (I) dans lesquels l'halogène est de l'iode radioactif peuvent être utilisés comme marqueurs ou comme agents thérapeutiques. Les dérivés halogénés de formule (1) dans laquelle X est un atome d'halogène non radioactif peuvent être utilisés dans des compositions pharmaceutiques, notamment comme β-bloquants.

Selon un premier mode de réalisation de l'invention, le dérivé répond à la formule :

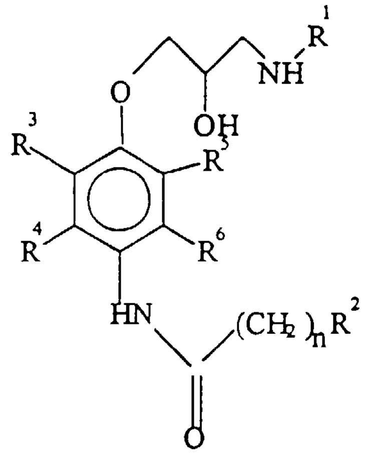

(II)

dans laquelle n, $R^1$, $R^3$, $R^4$, $R^5$ et $R^6$ sont tels que définis ci-dessus et $R^2$ représente CH=CHI.

A titre d'exemple de dérivés de ce type, on peut citer ceux pour lesquels n=0 ou 1, $R^1$ est le groupe isopropyle, $R^2$ représente CH=CHI et $R^3$, $R^4$, $R^5$ et $R^6$ sont des atomes d'hydrogènes.

Selon un second mode de réalisation de l'invention, le dérivé répond à la formule :

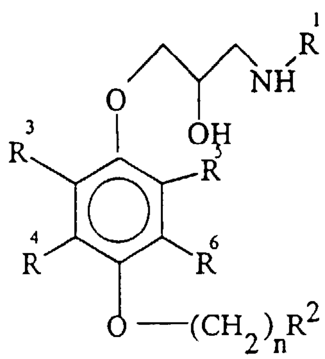

(III)

dans laquelle n, $R^1$, $R^3$, $R^4$, $R^5$ et $R^6$ sont tels que définis ci-dessus et $R^2$ représente $O(CH_2)_2I$, $O(CH_2)_2Cl$ ou $OCH_2$-CH=CHI.

A titre d'exemples de dérivés de ce type, on peut citer ceux pour lesquels n=2, $R^1$ est le groupe isopropyle, $R^2$ est le groupe O-$(CH_2)_2$-I, $O(CH_2)_2Cl$ ou $OCH_2CH$=CHI, et $R^3$, $R^4$, $R^5$ et $R^6$ sont des atomes d'hyrogène.

De préférence, dans les deux modes de réalisation de l'invention, $R^2$ est le groupe

- -CH=CHX,
- -C≡CX,
- -O-$CH_2CH$=$CH_X$, ou
- -O-$CH_2$-$CH_2X$

avec X représentant un atome d'un isotope radioactif de l'iode.

Les dérivés halogénés de l'invention sont très intéressants, car ils possèdent l'affinité élevée du practolol pour les récepteurs β1 adrénergiques, tout en ayant une lipophilie plus faible que celle des dérivés iodés du practolol connus et une meilleure stabilité in vivo que ces derniers.

Les dérivés halogénés de formule (II) dans laquelle $R^2$ représente -CH=CHX ou -C≡CX, avec X étant un atome d'halogène non radioactif peuvent être préparés par un procédé comprenant :

a) la réaction du 4-aminophénol avec un acide de formule $R^2$-$(CH_2)_n$-COOH pour obtenir un amide de formule :

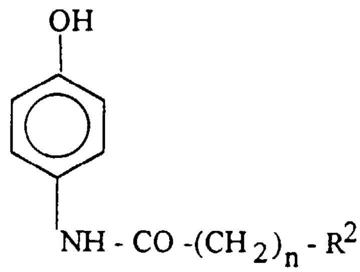

b) la glycidylation de l'amide ainsi obtenu par réaction avec le tosylate de glycidyle pour obtenir un époxyde de formule :

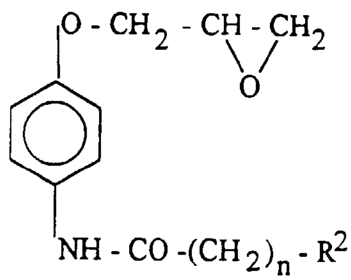

c) la réaction de l'époxyde avec une amine de formule $R^1NH_2$ pour obtenir le dérivé halogéné de formule (II).

Dans l'étape b), le tosylate de glycidyle peut être soit R, soit S, soit R et S.

Lorsqu'on veut obtenir un dérivé halogéné marqué par un isotope radioactif, le procédé comprend de plus une étape complémentaire d'échange de l'atome d'halogène X par le même halogène X radioactif.

Les techniques utilisées pour réaliser cet échange sont des techniques classiques.

Par exemple, dans le cas de l'iode, on peut réaliser l'échange à chaud avec de l'iodure radioactif, par exemple KI. On peut aussi obtenir une activité spécifique plus élevée, en réalisant par exemple un échange étain-iode sur un organostannique.

Les acides de formule $R^2$-$(CH_2)_n$COOH avec $R^2$ représentant -CH=CHX, peuvent être préparés par réaction d'un ester méthylique de formule :

$$CH\equiv C\text{-}(CH_2)_n\text{-}COOCH_3$$

avec l'hydrure de tributylétain pour former l'ester de formule :

$$(nC_4H_9)_3Sn\text{-}CH\text{=}CH\text{-}(CH_2)_n\text{-}COOCH_3,$$

suivie d'une halogénation par un agent halogénant approprié et d'une hydrolyse de l'ester halogéné obtenu.

Lorsque l'halogène est l'iode, on peut utiliser comme agent halogénant le monochlorure d'iode.

Les acides de formule $R^2$-$(CH_2)_n$-COOH avec $R^2$ représentant -C≡CX peuvent être préparés par réaction du complexe iode-morpholine sur l'ester de formule $CH\equiv C$-$(CH_2)_n$$COOCH_3$ suivie d'une hydrolyse.

Les dérivés halogénés de formule (I) avec $R^2$ représentant O-$(CH_2)_2$X peuvent être préparés par un procédé comprenant les étapes suivantes :

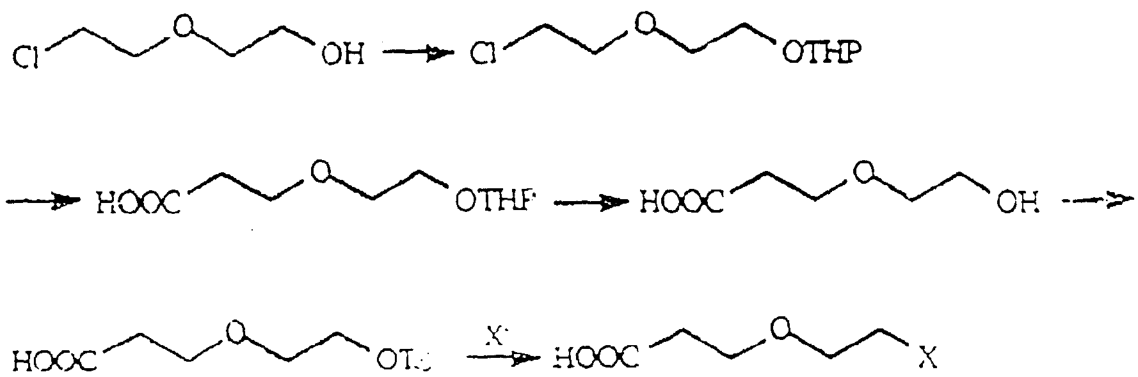


THP = tétrahydropyranne

Les dérivés halogénés de formule (II) avec $R_2$ représentant $OCH_2$-CH=CHX peuvent être préparés à partir du composé de formule :

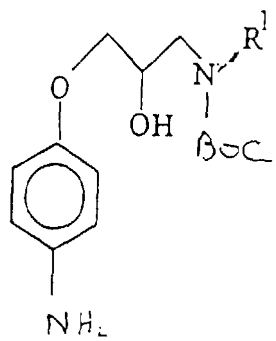

dans laquelle $R^1$ a la signification donnée ci-dessus et BOC représente le groupe butyloxycarbonyle, par réaction avec l'alcool de formule XCH=CHOH suivi d'une élimination du groupe protecteur BOC.

Ceci correspond au schéma réactionnel suivant pour le composé 15.

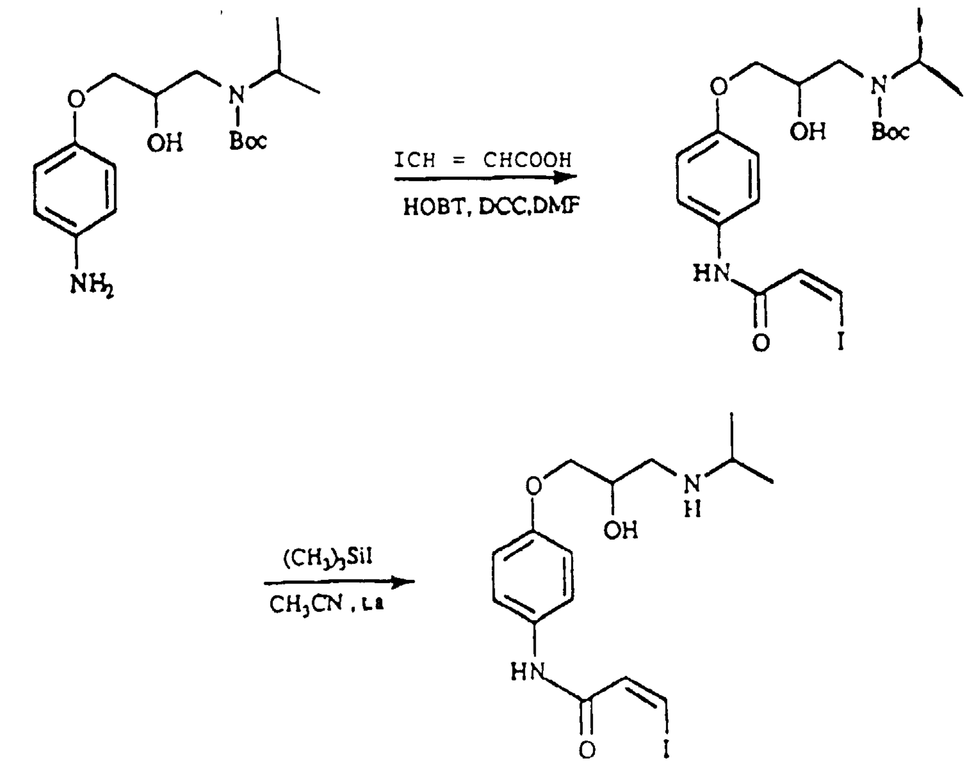


HOBT = hydroxybenzotriazole composé 15

L'acide ICH=CHCOOH peut être obtenu directement par action de l'acide iodhydrique sur l'acide propiolique et conduit dans les conditions utilisées à l'isomère Z uniquement. Ces résultats sont en accord avec le mécanisme d'addition proposé par BOWDEN et PRICE.

$$HC \equiv CHCOOH \xrightarrow[75°\,C,\,2h]{HI(57\%)} ICH = \underset{47\%(Z)}{CHCOOH}$$

Les dérivés halogénés de formule (III) avec $R^2$ représentant $O(CH_2)_2X$ peuvent être préparés par réaction d'un composé de formule :

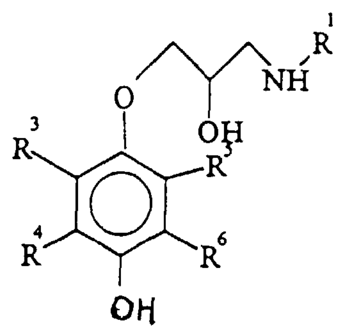

avec un composé de formule :

$$Cl(CH_2)_nOCH_2CH_2OH$$

Ainsi, le 1-{4-[2-(2-iodoéthoxy)ethoxy] phénoxy}-3-isopropyl-aminopropan-2-ol (chlorhydrate) peut être préparé à partir du prénaltérol ayant la structure suivante :

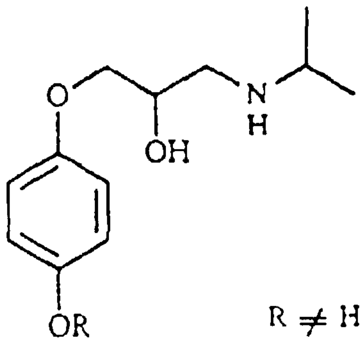

R ≠ H

Il peut être facilement obtenu en grande quantité selon le schéma de synthèse qui suit à partir du 4-benzyloxyphénol commercialisé. Le rendement de chacune des trois étapes est excellent.

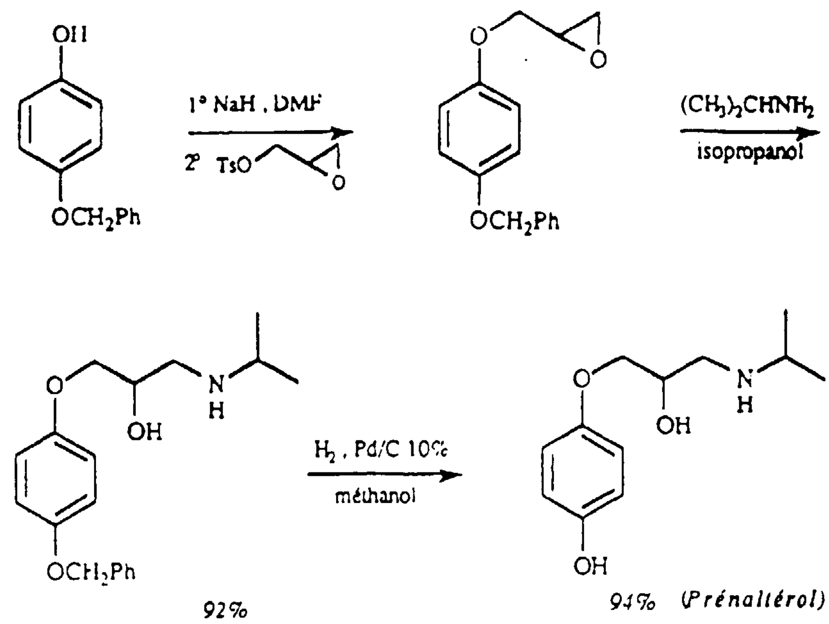


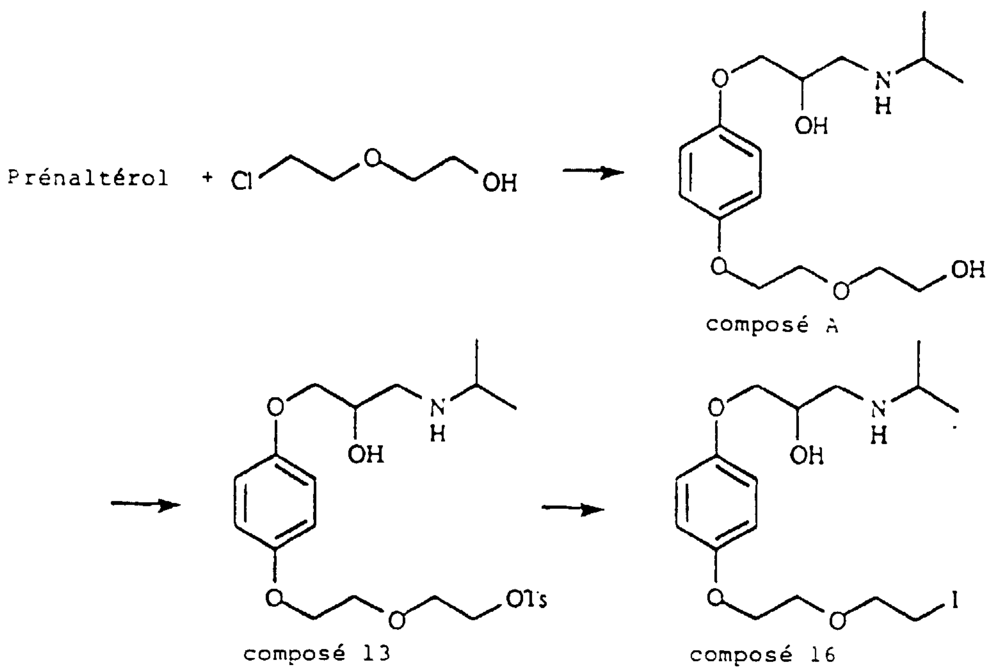
Prénaltérol +

composé A

composé 13

composé 16

On peut espérer que, lors de la tosylation de A pour obtenir B, il n'y aura que peu de réaction sur l'azote. Bien que les amines soient plus nucléoph les que les alcools, on a en effet ici concurrence entre une fonction alcool primaire et une fonction amine secondaire déjà bien encombrée, donc moins réactive. En fait, les résultats obtenus montrent qu'il n'en est rien : en effectuant la réaction à température ambiante la tosylation sur l'azote s'avère prépondérante (60% environ) . Le spectre de RMN du proton permet de le montrer facilement : en effet, les signaux des méthyles des groupes - $NSO_2C_6H_4CH_3$ et $OSO_2C_6H_4CH_3$ se situent à 2,40 et 2,16 ppm respectivement.

Le composé 16 peut aussi être préparé par le schéma qui suit, dans lequel on protège d'abord la fonction amine sous forme du carbamate.

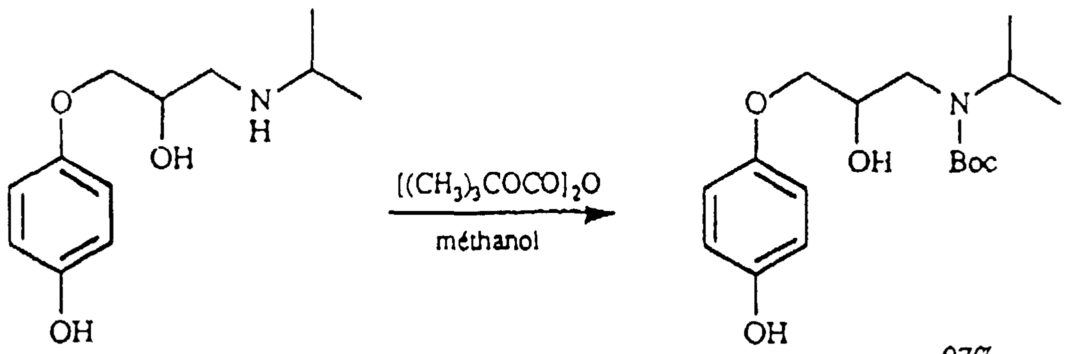


Prénaltérol

97%

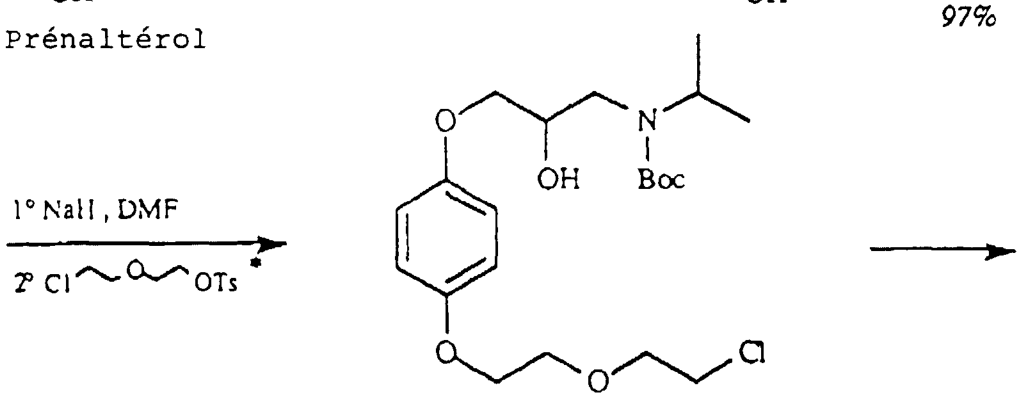


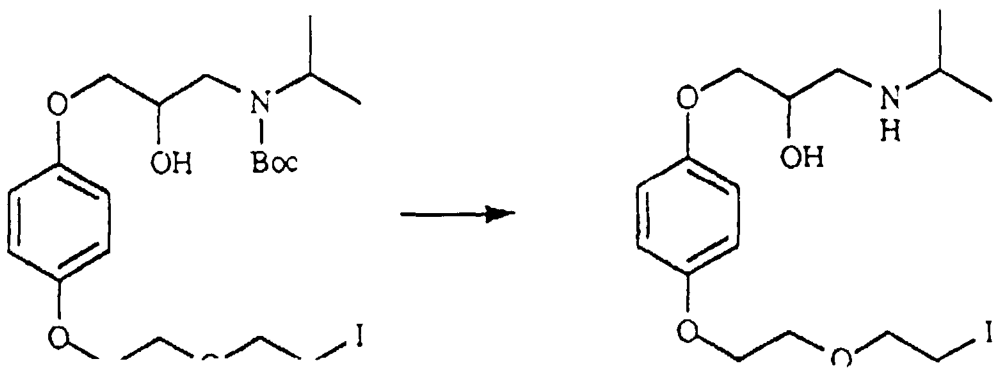

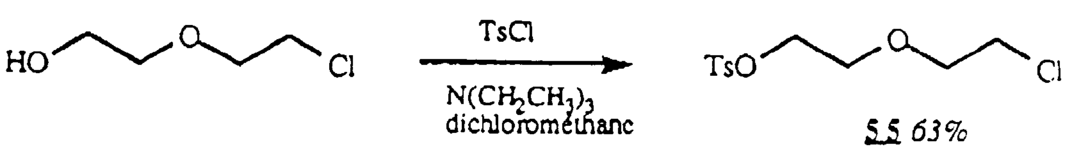


5.5 63%

Les dérivés de formule (III) avec $R^2$ représentant $OCH_2CH{=}CHX$ peuvent être préparés en suivant le schéma réactionnel qui suit et qui correspond à la préparation du composé 10, c'est-à-dire le 1-[4- (5-iodo-2-oxapent-4-ényloxy) phénoxy]-3-isopropylaminopropan-2-ol.

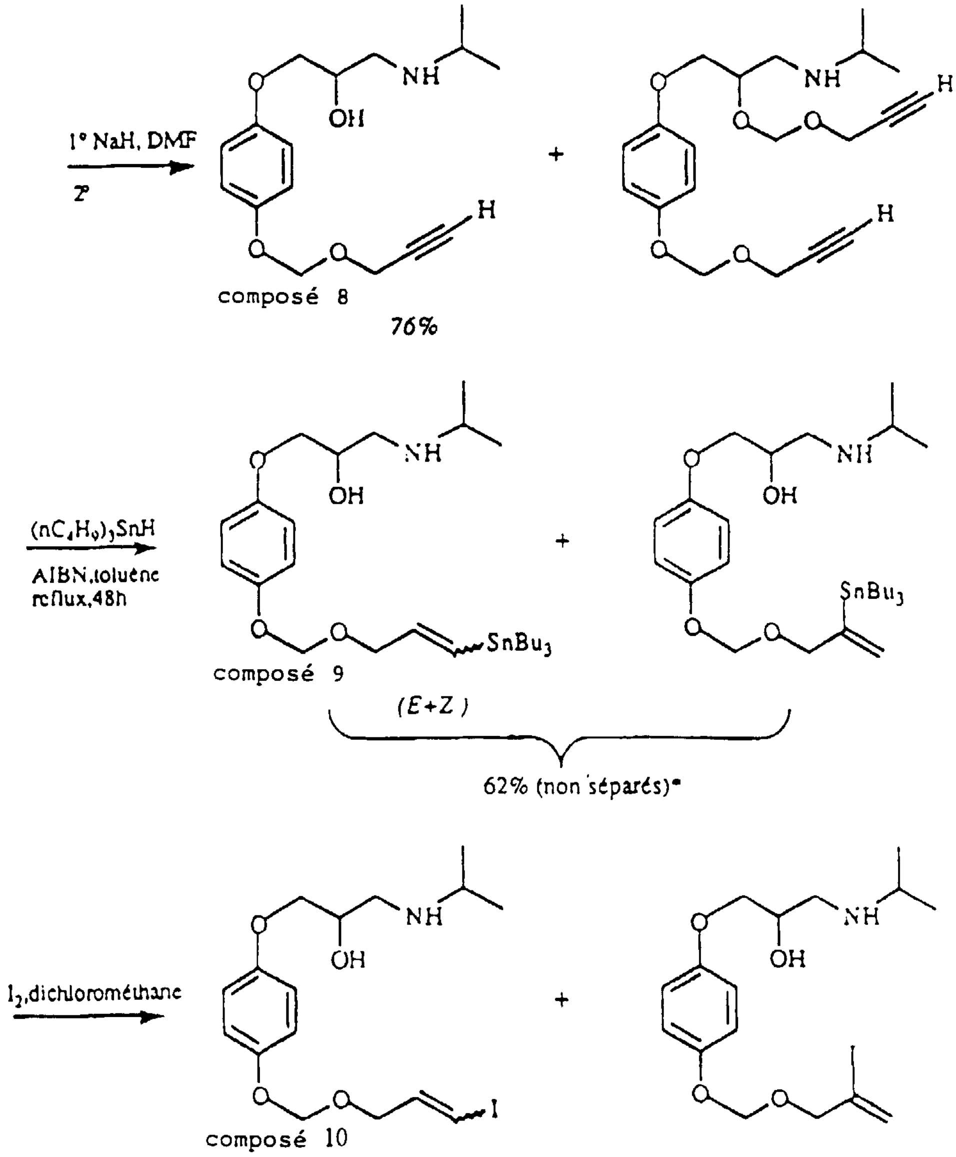


Une des meilleures méthodes d'obtention des iodures vinyliques consiste à passer par un dérivé stannique. Celui-ci subit facilement une destannylation en présence d'iode, de chlorure d'iode ou d'iodosuccinimide par exemple. La substitution électrophile qui se produit ne modifie pas la stéréochimie autour de la double liaison : l'halogène remplace simplement le groupe $R_3Sn$. Cette méthode d'halogénation est également applicable à la préparation de composés radioiodés à haute activité spécifique ; ceci s'avère particulièrement intéressant en permettant l'emploi de faibles doses lors des essais biologiques.

Les dérivés halogénés de l'invention sont plus intéressants que les dérivés halogénés décrits dans l'art antérieur, car ils présentent une bonne affinité pour les récepteurs β adrénergiques avec une spécificité élevée, une hydrophilie améliorée par rapport aux dérivés iodés du practolol comportant un cycle aromatique sur lequel est fixé l'atome d'iode, et une bonne stabilité in vivo en raison de la fixation de l'atome d'halogène sur un carbone insaturé ou en β d'un atome d'oxygène.

En raison de leur affinité pour les récepteurs β adrénergiques myocardiques ,les dérivés halogénés de l'invention peuvent être utilisés comme β bloquants lorsqu'ils comportent un atome d'halogène non radioactif, ou comme marqueurs, pour l'étude des récepteurs β adrénergiques in vivo, en vue par exemple de dénombrer ces récepteurs, lorsqu'ils comportent un isotope radioactif d'un halogène.

Dans ce dernier cas, on peut par exemple administrer un dérivé iodé conforme à l'invention, par injection intraveineuse, par exemple sous la forme de solution hydroalcoolique, puis réaliser l'examen au moyen d'une gamma caméra, environ 30 minutes après l'injection.

Pour ces applications en thérapeutique ou en diagnostic, on peut utiliser le dérivé halogéné de l'invention soit sous

la forme de mélanges (R, S), soit sous la forme d'énantiomère pur.

Les procédés de synthèse conduisent soit au mélange racémique, soit à l'un ou l'autre des énantiomères que l'on peut purifier par des techniques classiques.

D'autres caractéristiques et avantages de l'invention apparaîtront mieux à la lecture des exemples suivants, donnés bien entendu à titre illustratif et non limitatif.

Les exemples 1 à 6 illustrent la synthèse du 1-[4-(4-iodobut-3-énamido) phénoxy]-3-N-isopropylaminopropan-2-ol, conformément au schéma réactionnel suivant :

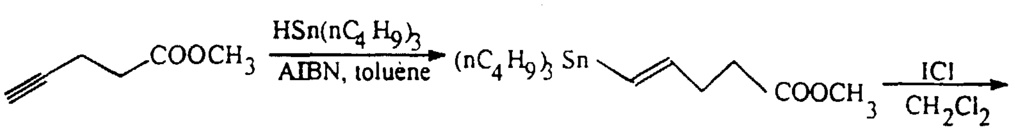


composé n° 1

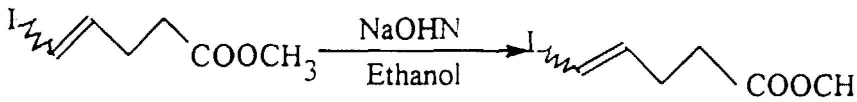


composé n° 2 (E/Z:80/20) composé n° 3

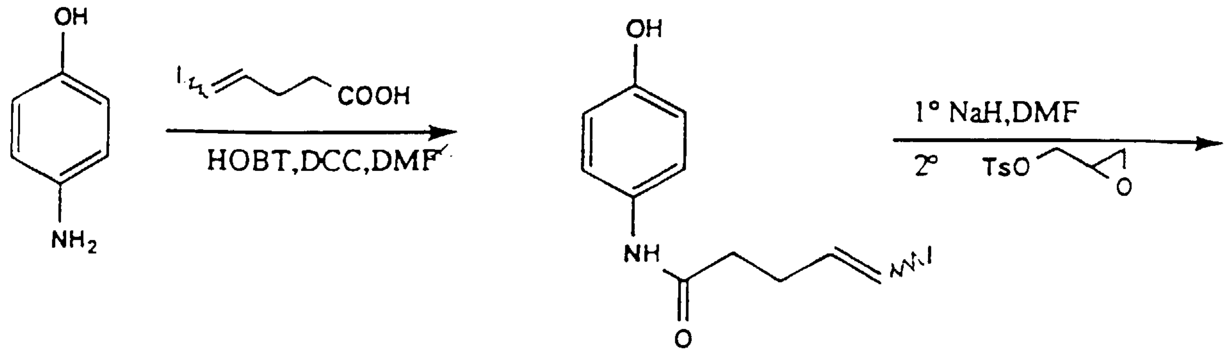


composé 4

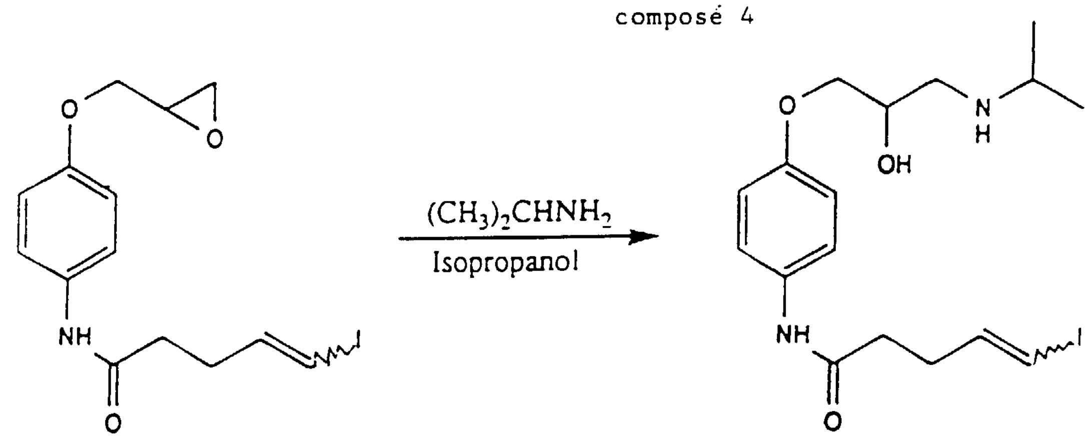


composé 5 composé 6

L'exemple 7 illustre la préparation du dérivé halogéné (composé n° 6) marqué à l'iode $^{123}I$.

Les exemples 8 à 12 illustrent les propriétés et les résultats obtenus avec le composé n° 6 de l'exemple 6, marqué avec $^{123}I$.

**Exemple 1 :** Préparation du 5-(tri-n-butylstannyl) pent-4-énoate de méthyle (composé n° 1)

On introduit dans un ballon de 250 ml sous atmosphère d'argon 120 ml de toluène, 2,24 g ($2.10^{-2}$ mol) de pent-4-ynoate de méthyle, 6,1 g($2,1.10^{-2}$ mol) d'hydrure de tributylétain et 300 mg d'azobis-isobutyronitrile. Le mélange est porté au reflux et l'évolution de la réaction suivie par infrarouge. Le toluène est éliminé à l'évaporateur rotatif au bout de 5 h. Le résidu (9 g) est passé rapidement sur colonne de silice (200 g) à l'aide du mélange éluant hexane-acétone (98/2). On obtient 7,40 g de composé n° 1 sous forme d'une huile incolore (rendement de 92 %). On peut également purifier le composé par distillation.

Les caractéristiques de ce composé sont les suivantes :

$Eb_{0,002}$=98-105°C.

RMN$^1$H-δ-$C\underline{H}_3CH_2CH_2C\underline{H}_2$:0,70-1,05,m(15H); $CH_3C\underline{H}_2C\underline{H}_2CH_2$:1,20-1,70,2m (12H) ; $C\underline{H}_2C\underline{H}_2CO$:2,35-2,46, m(4H) ; $C\underline{H}_3O$:3,67 et 3,68 ppm, 2s (3H) ; $SnC\underline{H}=C\underline{H}$:5,77-6,54, m(2H).

RMN$^{13}$C-δ-$\underline{C}H_2Sn$:9,31 et 10,13 ; $\underline{C}H_3CH_2$-:13,60 ; $CH_3\underline{C}H_2\underline{C}H_2$- et -$CO\underline{C}H_2\underline{C}H_2$- : 27,20-27,73-29,15-29,24-32,10-32,61-33,32-34,18 ; $\underline{C}H_3O$:51,26 ;=$\underline{C}HSn$:128,69 et 129,94 ; =$\underline{C}H-CH_2$-:146,39 et 146,66 ; C=O:173,32 et 173,64.

IR-ν(C=O):1740 $cm^{-1}$ ; ν(C=C):1605 $cm^{-1}$.

**Exemple 2 :** Préparation du 5-iodopent-4-énoate de méthyle (composé n° 2).

On introduit dans un ballon de 250 ml une solution de 8,31 g ($2,06.10^{-2}$ mol) du composé n° 1 dans 150 ml de $CH_2Cl_2$ et on abaisse la température à 0°C. On ajoute alors par une ampoule isobare une solution de 3,35 g ($2,06.10^{-2}$ mol) de monochlorure d'iode dans 50 ml de $CH_2Cl_2$. Lors de l'addition (goutte à goutte rapide) la solution de chlorure d'iode se décolore instantanément. Une chromatographie sur couche mince (CCM) de la solution jaune pâle obtenue, effectuée immédiatement après la fin de l'addition, montre l'absence de composé de départ (éluant : hexane-acétone 98/2 ; révélateur : UV, iode, réactif phosphomolybdique ; Rf:composé n° 1 = 0,42 et composé n° 2 = 0,26).

Le solvant est évaporé et le résidu (11,9 g) est passé rapidement sur une colonne de silice (500 g en deux fois) à l'aide du mélange éluant $CH_2Cl_2$-hexane (4/1). On obtient 4,10 g (83 % de composé n° 2, liquide incolore (rendement de 83 %).

En chromatographie gazeuse (CPG) sur colonne carbowax 20M (1,5 m ; 10 %), on note la présence de deux pics dans le rapport 19/81. Après séparation, le composé prépondérant (isomère E) présente les caractéristiques spectrales qui suivent.

IR - ν(=C-H):3060 $cm^{-1}$; ν(C=O):1740 $cm^{-1}$ ; ν (C=C):1610 $cm^{-1}$;δ'(=CH):945 $cm^{-1}$.

RMN$^1$H-δ-$C\underline{H}_2C\underline{H}_2CO$:2,30-2,51, m(4H); $C\underline{H}_3O$-:3,68,s(3H); $IC\underline{H}$=:6,08 et 6,15, d(1H)J=14,42Hz; $CH_2C\underline{H}$=:6,45-6,59, m(1H).

RMN$^{13}$C-δ-$\underline{C}H_2\underline{C}H_2$:31 et 32,50; $\underline{C}H_30$:51,62; $I\underline{C}H$=76,17; $CH_2\underline{C}H$=:143,99; C=O:172,56.

Le composé minoritaire (isomère Z) présente en RMN du proton les signaux suivants :

δ-$\underline{C}H_2\underline{C}H_2CO$:2,33-2,55, m(4H); $C\underline{H}_3O$:3,69, s(3H); $CH_2C\underline{H}$= et $IC\underline{H}$=:6,20-6,31 m et d(2H)J=7,56 Hz.

Dans le spectre de RMN du proton du mélange injecté en CPG, le rapport de l'intensité des signaux situés entre 6,20 et 6,31 ppm à celle des autres signaux situés entre 6,45-6,59 et à 6,08 et 6,15 ppm est 19/81. Le rapport des signaux situés à 3,69 et 3,68 ppm est àe 25/75.

**Exemple 3 :** Préparation de l'acide 5-iodopent-4-énoïque (composé n° 3)

On agite à température ambiante un mélange de 700 mg ($2,9.10^{-3}$ mol) du composé n° 2, 15 ml d'éthanol et 7,5 ml de soude N ($7,5.10^{-3}$ mol). Un prélèvement du milieu réactionnel effectué au bout de deux heures montre la disparition du composé n° 2 et la formation d'un seul produit (CCM-éluant:chloroforme-méthanol:9/1). On double alors le volume par addition d'eau, puis on abaisse le pH à 1-2 au moyen d'HCl 2N. On extrait à l'éther (4x20 ml), on lave la phase organique obtenue à l'eau (10 ml). Après séchage et élimination du solvant, on obtient 0,643 g (97,5 %) de composé n° 3 sous la forme d'un solide cireux.

Le rendement est de 97,5 %.

Les caractéristiques de ce composé sont les suivantes :

PF=62-73°C.

IR-ν(OH):3600-2000 $cm^{-1}$ ; ν(C=O):1705 $cm^{-1}$;ν(C=C):1605 $cm^{-1}$ ; δ'(=CH):940 $cm^{-1}$.

RMN$^1$ H-δ-$C\underline{H}_2$-$C\underline{H}_2$:2,31-2,51,m(4H); $IC\underline{H}$=(E):6,10 et 6,18, dt(0,8H)J=14,45 et 1,25Hz; $CH_2C\underline{H}$=(E):6,47-6,60 dt partiellement superposés (0,8H) J=14,43 et 6,65Hz. $IC\underline{H}$= et $CH_2C\underline{H}$=(Z)6,22-6,34, m et d (0,4H) J =7,43Hz. COOH : 10,00 s(H)

RMN$^{13}$C-δ-$\underline{C}H_2\underline{C}H_2$:29,76 et 32,21 (Z) 30,70 et 32,60 (E); $I\underline{C}H$=:76,58(E) et 84,19(Z); $CH_2\underline{C}H$=:138,71(Z) et 143,62 (E); $\underline{C}$=O:178,90(E) et 179,19(Z).

**Exemple 4 :** Préparation du 4-(4-iodobut-3-énamido)phénol (composé n° 4)

On dissout dans 20 ml de DMF anhydre 0,550 g ($2,33.10^{-3}$ mol) de 4-aminophénol, 0,500 g($2,2.10^{-3}$ mol) de composé 3, 0,310 g ($2,3.10^{-3}$ mol) d'hydrate de 1-hydroxybenzotriazole (HOBT) et 0,474 g ($2,3.10^{-3}$ mol) de dicyclohexylcarbodiimide (DCC) . Un précipité de dicyclohexylurée apparaît au bout de quelques heures dans la solution laissée à température ambiante. L'évolution de la réaction est suivie par CCM (chloroforme-acétate d'éthyle:3/7). Les composés de départ ont disparu après une vingtaine d'heures environ. On filtre le précipité puis on élimine le solvant à la pompe. Le résidu solide est repris par quelques millilitres d'acétate d'éthyle. Après filtration du solide non dissous, la phase organique est lavée à l'eau (4x20 ml) puis séchée et le solvant est éliminé. Une CPL sur silice permet d'obtenir 0,592 g du composé n° 4 (rendement de 80 %) sous la forme de solide blanc.

Les caractéristiques de ce composé sont les suivantes :

PF=146-147°C

RMN$^1$H(DMSO$d_6$)-δ-$C\underline{H}_2C\underline{H}_2$:2,20-2,40ppm,m (4H); IC$\underline{H}$=(E):6,21 et 6,28, d(0,8H)J=14,50Hz; IC$\underline{H}$=(Z),C$\underline{H}$=C$\underline{H}$-$CH_2$(E+Z):6,41-6,70,m et d(3,2H)J=8,79Hz; protons aromatiques: 7,29 et 7,34, d(2H)J=8,71Hz; N$\underline{H}$:9,12, s (1H); O$\underline{H}$: 9,62, s(1H).

RMN$^{13}$C(DMSO $d_6$)-δ-$\underline{C}H_2\underline{C}H_2$:32,93 et 36,28; I$\underline{C}$H=:76,33; $\underline{C}$H aromatique : 116,18 et 123,48; $\underline{C}$-NH:131,55; ICH=$\underline{C}$H: 145,96; $\underline{C}$-O:155,64; $\underline{C}$=O;172,90.

Anal.($C_{11}H_{12}INO_2$)C, H, I, N, O

**Exemple 5 :** Préparation du 1-[4-(4-iodobut-3-énamido)phénoxy]-2,3-époxypropane (composé n° 5)

On introduit, sous atmosphère d'argon, dans un tricol de 50 ml équipé d'un thermomètre, d'un réfrigérant et d'une ampoule de coulée isobare 42 mg ($3,30.10^{-3}$ mol) d'hydrure de sodium en dispersion à 60 % dans le pétrole. On lave deux fois (en agitant légèrement) avec quelques millilitres de pentane qu'on récupère à la seringue. On ajoute alors 5 ml de DMF anhydre. Après refroidissement de la suspension à 0°C, on introduit goutte à goutte une solution de 330 mg ($1,04.10^{-3}$ mol) du composé n° 4 dans 10 ml de DMF. Au bout de deux heures environ, le dégagement d'hydrogène a cessé. On ajoute alors en un quart d'heure une solution de 0,237 g ($1,04.10^{-3}$ mol) de tosylate de glycidyle racémique dans quelques millilitres de DMF. On laisse revenir le milieu réactionnel à température ambiante. La réaction est suivie par CCM (éluant : acétate d'éthyle-chloroforme 2/8) en opérant de la façon suivante. On retire à la seringue entre 100 et 200 µl de solution qu'on verse dans 1 ml d'eau. On extrait à l'acétate d'éthyle et on chromatographie la phase organique. Après disparition des composés de départ (24 h environ), on élimine le DMF à la pompe. Le résidu solide obtenu est repris par 50 ml d'eau et 50 ml d'acétate d'éthyle. La phase aqueuse est extraite à l'acétate d'éthyle (2x20 ml). Après le traitement habituel, on obtient 750 mg de produit brut qu'on purifie par CPL. On récupère finalement 310 mg de composé n° 5 sous la forme de solide blanc (rendement de 80 %).

PF=133-135°C

RMN$^1$H-δ-$C\underline{H}_2C\underline{H}_2$:2,35-2,51,m(4H);

$C\underline{H}_2$épox.:2,73-2,77,dd(1H)J=4,89 et 2,64Hz et 2,88-2,93, dd en partie superposé (1H) J=4, 16Hz; C$\underline{H}$épox.:3,31-3,38, m(1H); ArO$C\underline{H}_2$:3,87-3,96,dd(1H) J=11,05 et 5,69Hz et 4,17-4,24,dd (1H) J=11,05 et 3,06Hz; IC$\underline{H}$=(E):6,09 et 6,17, d (1H)J=14,33Hz; $CH_2C\underline{H}$=(E):6,49-6,59, m (1H); protons aromatiques:6,84-6,89-7,36 et 7,40 2d (2x2H)J=8,96Hz; N$\underline{H}$: 7,27, s(1H).

RMN$^{13}$C-δ-$\underline{C}H_2\underline{C}H_2$:31,65 et 35, 80; $\underline{C}H_2$ épox.:44,69; $\underline{C}$H épox.: 50,16; Ar O$\underline{C}H_2$: 69,04; I$\underline{C}$H=76,42; $\underline{C}$H aromatique : 115,00 et 121,87; $\underline{C}$-NH:131,52; $CH_2\underline{C}$H=:144,32; $\underline{C}$-O:156,00; $\underline{C}$=O:169,95.

Anal.($C_{14}H_{16}INO_3$)→C, H, N, O.

**Exemple 6 :** Préparation du 1-[4-(4-iodobut-3-énamido)phénoxy]-3-N-isopropyl aminopropan-2-ol racémique (composé n° 6)

On porte au reflux une solution de 130 mg ($3,5.10^{-4}$ mol) de composé 5 et de 126 mg ($2,1.10^{-3}$ mol) d'isopropylamine dans 15 ml d'isopropanol. La réaction - suivie par CCM (éluant:chloroforme-méthanol 1/9) - est terminée au bout de 6h. Après évaporation du solvant, le résidu est passé sur colonne de silice. On obtient 128 mg de composé n° 6 sous la forme de produit solide blanc (rendement de 85 %).

PF=156-158°C

RMN$^1$H(MeOD)-δ-$(C\underline{H}_3)_2C$:1,08-1,12,2d(6H)J=6,27Hz;

$C\underline{H}_2C\underline{H}_2$:2,35-2,50,m(4H); $C\underline{H}_A H_B$NH:2,61-2,71,dd(1H)J=12,0 et 8,2Hz; C$\underline{H}$NH-$CH_AH_B$:2,76-2,95, dd(1H)J=12,0 et 3,5Hz et sept (1H)J=6,3Hz; O$C\underline{H}_2C\underline{H}$OH:3,86-4,11,m(3H);IC$\underline{H}$=C$\underline{H}$(Z+E):6,19-6,66,m(2H); protons aromatiques: 6,88-6,92-7,39 et 7,44,2d(4H)J=9,0Hz.

RMN$^{13}$C(MeOD)-δ-$(\underline{C}H_3)_2C$:22,32 et 22,43; $\underline{C}H_2$=$\underline{C}$H:32,90 et 36,31;$\underline{C}H_2$NH$\underline{C}$H:49,95 et 50,77; O$\underline{C}H_2\underline{C}$HOH:69,71 et 72,16; I$\underline{C}$H=:76,36;CH aromatique:115,68 et 123,10; $\underline{C}$NH:132,95; ICH=$\underline{C}$H:145,97; C-O:157,09; $\underline{C}$=O:172,65.

Anal. ($C_{17}H_{25}IN_2O_3$)C,H,I,N,O,

**Exemple 7 :** Marquage du composé n° 6 à l'iode 123.

On réalise l'échange sur 4 mg du composé n° 6 en utilisant 1 ml d'acétone et 4 µg de $K^{123}I$, à 170°C pendant 2 heures.

On obtient ainsi un composé radioactif stable qui présente les coefficients de partage octanol-eau à la température ambiante donnés dans le tableau 1 qui suit.

A titre comparatif, on a donné également dans ce tableau 1 les coefficients de partage obtenus dans les mêmes conditions avec des β-bloquants connus :

- le propranolol marqué au tritium, et
- le CGP 12177 marqué au tritium.

TABLEAU 1 :

| COEFFICIENT DE PARTAGE OCTANOL-EAU | | |
|---|---|---|
| | pH=4,2 | pH=7,4 |
| [$^{123}$I]-composé 6 | 0,253 | 4,086 |
| [$^{3}$H]-propranolol | 0,3 | 11,3 |
| [$^{3}$H]-CGP 12177 | 0,0088 | 0,18 |

Le composé 6 est donc moins hydrophobe que le propranolol.

**Exemple 8**

Dans cet exemple, on teste l'affinité du composé 6 marqué avec $^{123}$I vis-à-vis des récepteurs β adrénergiques.

On étudie sur cardiomyocytes de rats nouveau-nés la capacité du composé 6 à déplacer le [$^{3}$H]CGP 12177 préalablement fixé sur les récepteurs.

A la dose de $5.10^{-9}$M et après 30 minutes d'incubation, le [$^{3}$H]CGP 12177 occupe tous les récepteurs β adrénergiques des cardiomyocytes.

La concentration du propranolol nécessaire pour inhiber 50 % (CI 50) de cette fixation est de $1,9.10^{-7}$M. la CI50 de l'atenolol (β bloquant hydrophile) est de $1,9.10^{-5}$M.

Le composé 6 a une CI50 de $2,0.10^{-6}$M.

Ceci signifie qu'il est capable de déplacer le CGP 12177 préalablement fixé sur les récepteurs avec une affinité comprise entre celle du propranolol et celle de l'atenolol.

**Exemple 9 :**

Dans cet exemple, on teste la propriété pharmacologique antagoniste du composé 6 vis-à-vis des récepteurs β adrénergiques.

L'expérience est conduite sur coeur de rat isolé et perfusé selon la technique de Langendorff.

Après une préperfusion de 25 minutes avec une solution de Krebs-Henseleit, le coeur est perfusé avec de l'isoprénaline à la concentration de $10^{-8}$M pendant 10 minutes ; puis avec une solution d'isoprénaline à $10^{-8}$M associée à la molécule à tester à la concentration de $10^{-6}$M.

A ces concentrations, la contractilité du coeur, appréciée par la mesure de la dP/dt max, augmente sous isoprénaline seule de 254 % par rapport à la préperfusion, puis revient à la valeur basale lorsque du propranolol est perfusé à $10^{-6}$ M.

Si la molécule à tester diminue la dP/dt max en présence d'isoprénaline, on pourra affirmer que cette molécule se comporte comme un antagoniste de l'isoprénaline vis-à-vis des récepteurs β adrénergiques.

Les résultats obtenus avec le composé 6 sont donnés dans le tableau 2 qui suit.

TABLEAU 2

| DP/dt max (mmHg/min) | Préperfusion (25$^e$ min) | Isoprénaline (10$^e$ min) | Isoprénaline + molécule (10$^e$+8 min) |
|---|---|---|---|
| Isoprenaline ($10^{-8}$M) (n=6) | 715±325 | 2535±1233 | 2398±1088 |
| Isoprenaline ($10^{-8}$M) +propranolol ($10^{-6}$M) (n=6) | 1009±422 | 2973±867 | 814±240 * |
| Isoprenaline ($10^{-8}$M) +composé 6($10^{-6}$M) (n=6) | 1256±206 | 2827±715 | 973±240 * |

*p< 0,01/isoprénaline 10+8min

Dans ce tableau, n représente le nombre d'expériences.

Les résultats du tableau 2 montrent que le composé 6 antagonise complètement l'action de l'isoprénaline comme le propranolol.

**Exemple 10**

Dans cet exemple, on teste chez la souris les propriétés du [$^{123}$I] - composé 6 comme traceur radioactif pour l'examen du myocarde.

Injection chez la souris du composé 6 [$^{123}$I] et comparaison avec [$^3$H]CGP 12177

Après injection de $500.10^{-12}$ mol de [$^{123}$I]-composé 6, les animaux sont sacrifiés et les organes d'intérêt récupérés et comptés. Le tableau 3 qui suit reporte les valeurs de la radioactivité trouvée dans les organes ou liquides biologiques exprimées en pourcentage de la dose injectée par gramme d'organe.

TABLEAU 3 :

| [$^{123}$] composé 6 ($500.10^{-12}$ mol) | | | | | |
|---|---|---|---|---|---|
| **TEMPS** | 15 sec | 2 min | 5 min | 10 min | 15 min |
| **COEUR** | 14±3,5 | 17±2 | 11,3±2 | 6,4±0,6 | 5±0,5 |
| **POUMON** | 42±7 | 28±3,5 | 16±1,5 | 13±2 | 12±0,5 |
| **SANG/ml** | 19,6±2,8 | 2,8±0,2 | 3,1±0,4 | 2,6±0,3 | 2,8±0,5 |

Dans le tableau 4 qui suit, on a donné à titre comparatif les résultats obtenus lorsqu'on injecte $329.10^{-12}$ mol de [$^3$H]CGP 12177.

TABLEAU 4 :

| [$^3$H]CGP12177 ($329.10^{-12}$ mol) | | | | |
|---|---|---|---|---|
| **TEMPS** | 1 min | 2 min | 5 min | 10 min |
| **COEUR** | 6,8±4 | 6,3±0,3 | 5,9±0,8 | 5,3±0,2 |
| **POUMON** | 26,3±2,1 | 31,6±6,8 | 29,7±5,8 | 29±5,1 |

**Exemple 11**

Dans cet exemple, on étudie la captation myocardique du [$^{123}$I]-composé 6 chez le chien.

Dans ce but, on injecte 2,2 mCi de [$^{123}$I]-composé 6, soit 3,73 mg de composé 6.

Après injection du traceur, le devenir de la radioactivité est suivi par détection externe avec une gamma-caméra, pendant 60 minutes.

Le système informatique du traitement de l'image permet d'apprécier la radioactivité présente dans des zones préalablement sélectionnées.

La mesure de cette activité est exprimée en cpm/pixel.mCi.

Les résultats obtenus sont donnés dans le tableau 5 qui suit.

La radioactivité présente dans le sang est mesurée par prélèvement et comptage d'échantillons. Les résultats sont donnés dans le tableau 6 qui suit.

**Exemple 12**

Dans cet exemple, on étudie également la captation myocardique du [$^{123}$I]-composé 6 chez le chien en injectant simultanément 2,3 mCi de [$^{123}$I]-composé 6 (3 mg de composé 6) et 30 mg de timolol par voie intraveineuse.

On mesure ensuite l'activité comme dans l'exemple 11. Les résultats obtenus sont donnés dans les tableaux 7 et 8 qui suivent.

TABLEAU 5

| TEMPS EN MINUTES | 5 | 10 | 15 | 20 | 25 | 30 | 35 | 40 | 45 | 50 | 55 | 60 |
|---|---|---|---|---|---|---|---|---|---|---|---|---|
| Activité coeur (coup/pixel/min/mCi) | 70 | 67 | 64 | 62 | 60 | 58 | 56 | 56 | 52 | 51 | 48 | 49 |
| Activité poumon (coup/pixel/min/mCi) | 48 | 41 | 41 | 40 | 39 | 39 | 40 | 38 | 38 | 37 | 37 | 38 |
| Activité Bruit de Fond (Coup/pixel/min/mCi) | 34 | 33 | 33 | 34 | 34 | 34 | 34 | 35 | 34 | 36 | 35 | 35 |
| Rapport coeur/poumon | 1,46 | 1,63 | 1,56 | 1,55 | 1,53 | 1,49 | 1,40 | 1,47 | 1,37 | 1,37 | 1,30 | 1,29 |

TABLEAU 6 : ACTIVITE SANGUINE

| TEMPS EN MINUTES | 2 | 4 | 8 | 15 | 30 | 45 | 60 |
|---|---|---|---|---|---|---|---|
| Activité sanguine (% dose inj./mlx$10^{-3}$) | 11 | 4,7 | 2,8 | 2,4 | 2,5 | 2,7 | 3 |

TABLEAU 7 : [$^{123}$I]-composé 6 (2,3mCi) + Timolol (30 mg en I.V.)

| TEMPS EN MINUTES | 5 | 10 | 15 | 20 | 25 | 30 | 35 | 40 | 45 | 50 | 55 | 60 |
|---|---|---|---|---|---|---|---|---|---|---|---|---|
| Activité coeur (coup/pixel/min/mCi) | 50 | 45 | 40 | 38 | 34 | 34 | 32 | 30 | 30 | 29 | 29 | 28 |
| Activité poumon (coup/pixel/min/mCi) | 32 | 28 | 26 | 26 | 25 | 26 | 26 | 26 | 26 | 25 | 25 | 25 |
| Activité Bruit de fond (Coup/pixel/min/mCi) | 25 | 25 | 24 | 24 | 23 | 23 | 24 | 24 | 24 | 24 | 23 | 23 |
| Rapport coeur/poumon | 1,56 | 1,61 | 1,56 | 1,46 | 1,36 | 1,31 | 1,23 | 1,15 | 1,15 | 1,16 | 1,16 | 1,12 |

TABLEAU 8 : Activité sanguine

| TEMPS EN MINUTES | 2 | 4 | 8 | 15 | 30 | 45 | 60 |
|---|---|---|---|---|---|---|---|
| Activité sanguine (% dose inj./mlx$10^{-3}$) | 5,2 | 3 | 3 | 2,5 | 2,1 | 2,4 | 2,5 |

Les résultats du tableau 7 montrent que le bruit de fond représente 49 % de la fixation totale cardiaque à 5 min et 71 % à la 60$^{ème}$ minute. Pour mémoire, le bruit de fond d'un thallium à l'effort est de 29 % par rapport à la fixation cardiaque et après réinjection de 53 %.

Dans le tableau 9 qui suit, on a reporté les résultats de fixation des tableaux 5 et 7 pour apprécier la fixation non spécifique cardiaque.

TABLEAU 9 :

| Fixation non spécifique cardiaque (Activité en coup/pixel/min/mCi) | | | | | | |
|---|---|---|---|---|---|---|
| **TEMPS EN MINUTES** | **5** | **10** | **20** | **30** | **45** | **60** |
| Fixation totale (Act.Coeur-BF) | 36 | 34 | 28 | 24 | 18 | 14 |
| Fixation non spécifique (Act.Coeur-BF sous timolol | 25 | 20 | 14 | 11 | 6 | 5 |

TABLEAU 9 : (suite)

| Fixation non spécifique cardiaque (Activité en coup/pixel/min/mCi) | | | | | | |
|---|---|---|---|---|---|---|
| **TEMPS EN MINUTES** | **5** | **10** | **20** | **30** | **45** | **60** |
| % Fixation non spécifique | 69% | 59% | 50% | 46% | 33% | 36% |

Les exemples 8 à 12 montrent que le composé 6 marqué par $^{123}I$ est un marqueur des récepteurs β adrénergiques dont l'affinité est proche de celle du propranolol, mais dont l'hydrophobie est moindre. Ce composé antagonise l'effet de l'isoprénaline sur coeur isolé et perfusé de rat de manière identique au propranolol.

Après injection du composé 6 in vivo, chez le chien, l'activité cardiaque est constante à partir de la 45 ème minute. L'injection d'une faible quantité de timolol entraîne une diminution de l'activité cardiaque détectée de 60 à 67 % à partir de la 45ème minute. Ceci témoigne d'une fixation cardiaque spécifique importante.

Aussi, en augmentant la quantité de timolol injectée ainsi que l'activité spécifique de ce radioligand, on pourra diminuer l'importance de la fixation cardiaque non spécifique et ainsi apprécier la quantité de récepteurs β adrénergiques myocardiques, in vivo, par simple détection externe de la radioactivité cardiaque.

Les composés de l'invention sont donc très intéressants comme marqueurs des récepteurs β -adrénergiques.

**Exemple 13 : Préparation du 3-(chlorométhoxy)-prop-1-yne (composé 7)**

On introduit dans un tricol de 250 ml muni d'un réfrigérant, d'un thermomètre et d'une arrivée de gaz 56 g (1 mol) d'alcool propargylique et 30 g (0,33 mol) de trioxane. La température de l'ensemble est abaissée à -25°C par un bain acétone-neige carbonique. On place, par ailleurs, sur le plateau d'une balance une bouteille de HCl gazeux qu'on relie au ballon par l'intermédiaire d'une fiole de garde. On introduit alors bulle à bulle dans le tricol 50 g de chlorure d'hydrogène à raison d'un gramme par minute. Le mélange prend une teinte rosée et en fin d'addition on observe la présence de deux phases dans le ballon réactionnel. La phase supérieure est récupérée et séchée sur $CaCl_2$. L'excès de gaz est chassé sous pression réduite à température ambiante. Après distillation, on obtient 70 g (67%) de composé 7.

$Eb_{15}$=30°C.

IR - υ(=C-H) : 3280 $cm^{-1}$ ; υ(C≡C) : 2105 $cm^{-1}$ ; υ (C-O) : 1050 $cm^{-1}$.

RMN $^1H$ (AC200) ($CDCl_3$)-δ-≡CH : 2,54, t (1H), J=2,4 Hz, OC<u>H</u>$_2$C≡CH ; 4,38 et 4,40, d (2H), J=2,4 Hz, ClC<u>H</u>$_2$O : 5,59, s (2H).

RMN $^{13}C$ (AC200) ($CDCl_3$)-δ-<u>C</u>H$_2$C=5,55,80 ; ≡<u>C</u>H : 75,96, <u>C</u>H$_2$C≡C ; 76,99 ; Cl<u>C</u>H$_2$O : 79,80.

**Exemple 14 : Préparation du 1-[4-(-oxapent-4-ynyloxy) phénoly]-3-isopropylaminopropan-2-ol racémique : (composé 8)**

A partir de 4 g (1,78.$10^{-2}$ mol) de prénaltérol racémique, de formule :

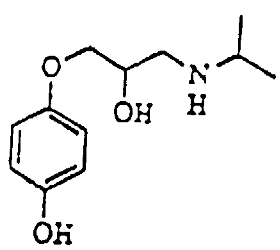

0,832 g (2,1.$10^{-2}$ mol) de NaH à 60% et 1,86 g (1,79.$10^{-2}$ mol) de composé 7, on obtient, après CPL, 3,96 g (76%) de composé 8 sous forme d'une poudre blanche.

P.F : 53-54°C.

RMN $^1H$ (AC200) ($CDCl_3$)-δ-($CH_3$)$_2$C : centré sur 1,5, d(6H), J=6,5Hz ; N<u>H</u> et O<u>H</u> : 2,35, s(2H) ; C≡C-<u>H</u> : 2,46, t-1H), J=2,4 Hz ; C<u>H</u>$_2$NHC<u>H</u> : 2,65-2,91, m(3H) ; OC<u>H</u>$_2$C<u>H</u>OH : 3,87-4,05, m(3H) ; C<u>H</u>$_2$C≡CH : 4,33, d(2H), J=2,4 Hz ; OC<u>H</u>$_2$O : 5,24, s(2H) ; CH arom. : 6,80-7,02, 2d(4H), J=9,3 Hz.

RMN $^{13}C$ (AC200) ($CDCl_3$)-δ-(<u>C</u>H$_3$)$_2$C : 23,06 et 23,16 ; C<u>H</u>$_2$NHC<u>H</u> : 48,86 et 49,26 ; C<u>H</u>$_2$C≡CH : 54,63 ; <u>C</u>HOH : 68,54 ; O<u>C</u>H$_2$: 71,13 ; C=<u>C</u>-H : 74,87 ; <u>C</u>≡CH : 78,77 ; OC<u>H</u>$_2$O : 91,86 ; <u>C</u>H arom. : 115,42 et 117,47 ; <u>C</u>O : 151,36 et 153,99.

| Analyse | C | H | N | O |
|---|---|---|---|---|
| calculée | 65,50 | 7,90 | 4,77 | 21,82 |
| trouvée | 65,03 | 7,92 | 4,99 | 21,44 |

**Exemple 15 : Préparation de 1-[4-(5-tri-n-butylstannyl-2-oxapent-4-ényloxy)phénoxy]-3-isopropyl-aminopropan-2-ol racémique : (composé 9)**

On introduit dans un ballon, sous atmosphère d'argon, 1,63 g ($5,57.10^{-3}$ mol) de composé 8, 3,2 g ($1,1.10^{-2}$ mol) d'hydrure de tributylétain, environ 200 mg d'AIBN et 10 ml de toluène anhydre. On chauffe à reflux et suit l'évolution de la réaction par CCM (chloroforme-méthanol-ammoniaque à 28% : 95/4,9/0,1). L'addition d'AIBN est répétée quand la réaction semble ne plus évoluer. Le toluène est éliminé à l'évaporateur rotatif au bout de 2 jours et le résidu chromatographié sur silice. Une première élution réalisée à l'aide d'éther ou d'acétate d'éthyle en présence d'ammoniaque (0,1%) permet d'éliminer l'excès d'hydrure de tributylétain. L'éluant utilisé en CCM permet alors de récupérer 1,99 g de composé 9 (62%) sous forme d'une huile jaune épaisse.

RMN $^1H$(AC200) ($CDCl_3$)-δ-$C\underline{H}_3CH_2CH_2C\underline{H}_2$ : 0,70-0,95, m (15H) ; $(C\underline{H}_3)_2C$ et $CH_3C\underline{H}_2C\underline{H}_2$ : 1,05-1,70, m (18H) ; $C\underline{H}_2NHC\underline{H}$ : 2,80-3,20, m (3H) ; $OC\underline{H}_2$-CH= et$C\underline{H}_2OC\underline{H}OH$ : 3,85-4,40, m(5H) ; N$\underline{H}$ et O$\underline{H}$ : 4,97, s (2H) ; $OC\underline{H}_2O$ : 5,14 et 5,16, 2s (2H au total) ; Sn-CH=$C\underline{H}_2$ : 5,07-5,16, m(0,4H environ) ; SnC$\underline{H}$=C$\underline{H}$ (Z et E) : 5, 85-6, 30 et 6,55-6,67, m (1, 6H au total) ; on peut distinguer entre 5,96 et 6,30 ppm un doublet de triplet et un doublet (isomère E-1H environ) avec J=19,1Hz et 4,9 Hz. Le signal situé entre 6,55 et 6,667 ppm permet de calculer deux des couplages du système éthylénique de l'isomère Z : 12,9 Hz et 5,9 Hz ; C$\underline{H}$ arom. : 6,80-7,04, 2d(4H), J=9,2 Hz.

RMN $^{13}C$ (AC200) ($CDCl_3$)-δ-$\underline{C}H_2Sn$ : 9,37 et 9,48 ; $\underline{C}H_3CH_2$ : 13,64 ; $(\underline{C}H_3)_2C$ : 20,96 et 21,06 ; $CH_3\underline{C}H_2\underline{C}H_2$ : 27,21-27,29-29,01-29,16 ; $\underline{C}H_2NH$ : 48,75, $\underline{C}HNH$ : 50, 15 ; $\underline{C}HOH$ : 67,10 ; $\underline{C}H_2CHOH$ : 70,61; $O\underline{C}H_2CH$=75,17 ; $O\underline{C}H_2O$ : 93,06 ; $\underline{C}H$ arom. : 115,33 et 117,41 ; Sn$\underline{C}$H= : 131,77 ; SnCH=$\underline{C}$H : 143,41 ; $\underline{C}O$ : 151,50 et 153,42.

| Analyse | C | H | N | O | Sn |
|---|---|---|---|---|---|
| calculée | 57,03 | 8,57 | 2,40 | 10,97 | 20,36 |
| trouvée | 56,56 | 8,99 | 2,34 | 10,33 | 20,27 |

**Exemple 16 : Préparation du 1-[4-(5-iodo-2-oxapent-4-ényloxy)phénoxy]3-isopropylaminopropan-2-ol racémique : (composé 10)**

On ajoute un équivalent d'iode à une solution d'un gramme ($2,02.10^{-3}$ mol) de composé 9 dans 5 ml de dichlorométhane. Le mélange réactionnel est agité à température ambiante. L'évolution de la réaction est suivie par CCM (éluant : chloroforme-méthanol-ammoniaque à 28% : 95/4,9/0,1). Une triple migration permet de bien séparer les composés de départ et d'arrivée. Aprés élimination du solvant, une première élution à l'aide d'acétate d'éthyle permet d'éliminer les dérivés stanniques. L'éluant utilisé pour la CCM permet ensuite d'obtenir le composé 10. Un double passage fournit 0,36 g (43%) d'un produit huileux légèrement jaune et de bonne pureté.

RMN $^1H$ (AC200) ($CDCl_3$)-δ-$(C\underline{H}_3)_2C$ : 1,02 et 1,05, d(6H), J=6,3 Hz ; N$\underline{H}$ et O$\underline{H}$ : 2,47, s (2H) ; $C\underline{H}_2NHC\underline{H}$ : 2,61-2,87, m(3H) ; $\underline{C}H_2CHOH$ : 3,86-4,02, m(3H) ; $OC\underline{H}_2CH$= : 4,04-4,34, m(2H) ; $OC\underline{H}_2O$ : 5,09-5,12 et 5,13, 3s (2H) ; ICH=$C\underline{H}_2$ : 5, 92, m (0,4H environ) ; IC$\underline{H}$=C$\underline{H}$ (Z et E) : 6,36-6,47 et 6,54-6,68, m (1,6H au total) ; C$\underline{H}$ arom. : 6,75-6,96, m(4H).

RMN $^{13}C$ (AC200) ($CDCl_3$)-δ-$(\underline{C}H_3)_2C$ : 22,64 et 22,96 ; $\underline{C}HNH$ : 49,01 ; $\underline{C}H_2NH$ : 49,19 ; $\underline{C}HOH$ : 68,42 ; $\underline{C}H_2CHOH$ : 71,09 ; $O\underline{C}H_2$=CH : 75,10 ; I$\underline{C}$H= 79,31 ; $O\underline{C}H_2O$ : 92,61 ; $\underline{C}H$ arom. : 115,47 et 117,51 ; ICH=$\underline{C}$H : 141,49 ; $\underline{C}$-O : 151,42 et 153,94.

| Analyse | C | H | N |
|---|---|---|---|
| calculée | 45,34 | 5,70 | 3,32 |
| trouvée | 45,67 | 5,78 | 3,11 |

**Exemple 17 : Préparation de 1-[4-(amino) phénoxy]-3-N-isopropylaminopropan-2-ol racémique : (composé 11)**

9,4 g ($3,53.10^{-2}$ mol) de practolol sont dissous dans 200 ml de HCl 2N. On porte au reflux et suit l'hydrolyse par CCM (éluant : ammoniaque à 28% - méthanol : 4/100). Lorsque la réaction est terminée, on ajuste le pH à 8 en ajoutant avec précaution $HNaCO_3$ solide. On évapore à sec sous pression réduite et extrait par l'acétate d'éthyle le résidu

solide placé dans un soxhlet. On récupère 7,75 g de produit brut. Ce dernier est purifié par CPL rapide sur silice (éluant : ammoniaque à 28% - méthanol : 1/100). On obtient 6,68 g (84%) de solide qui brunit rapidement. P. F=115-118°C.

RMN $^1$H (AC200) ($CDCl_3$)-δ-($C\underline{H}_3$)$_2$C : 1,06 et 1,09, d(6H), J=6,2 Hz ; $C\underline{H}_2N\underline{H}C\underline{H}(CH_3)_2$, $CHO\underline{H}$, $N\underline{H}_2$ : 2,50-3,20, m et s (très large)(7H) ; $OC\underline{H}_2C\underline{H}OH$ : 3,86-4,05, m(3H) ; C$\underline{H}$ arom. : 6,59-6,64-6,72 et 6,77, d(4H), J=9,7 Hz.

RMN $^{13}$C (AC200) ($CDCl_3$)-δ-($\underline{C}H_3$)$_2$C : 22,94 ; $\underline{C}H_2NH\underline{C}H$ : 48,81 et 49,37 ; $\underline{C}H_2\underline{C}HOH$: 68,56 et 71,34 ; $\underline{C}H$ arom. : 115,66 et 116,31 ; $\underline{C}NH_2$ : 140,18 ; $\underline{C}O$ : 151,81.

**Exemple 18 : Préparation du 1-[4-(amino)phénoxy]-3-N-isopropyl-N-tertbutoxycarbonylaminopropan-2-ol racémique : (composé 12)**

On porte à ébullition dans un ballon muni d'un appareil de Dean et Stark une solution de 673 mg ($3.10^{-3}$ mol) de composé 11, 323 mg ($3{,}04.10^{-3}$ mol) de benzaldéhyde (conservé sous atmosphère d'argon) dans 35 ml de benzène anhydre. Un dépôt solide translucide apparaît assez rapidement. On suit l'évolution de la réaction par CCM (éluant : ammoniaque à 28% - méthanol : 4/100). Un prélèvement effectué au bout de 3 heures montre par RMN la présence du proton de l'imine à 8,5 ppm et la disparition de celui de l'aldéhyde à 10 ppm. Après refroidissement, on ajoute 660 mg ($3{,}02.10^{-3}$ mol) de dicarbonate de tertiobutyle et agite pendant 24h. La phase liquide est alors évaporée sous pression réduite et on obtient 1,1 g (89%) de résidu très visqueux. Ce dernier présente un Rf de 0,42 dans le système étant chloroforme-méthanol (97,5/2,5). La tache correspondant à ce produit peut être mise en évidence à l'aide d'une solution d'acide phosphomolybdique dans l'éthanol (coloration grise d'intensité moyenne). Après passage sur une colonne de silice (80 g) avec une vitesse d'élution lente (1 ml/5 min), on obtient 690 mg d'un produit très visqueux possédant un Rf de 0,25 et qui donne par révélation avec le même réactif une tache noire très intense. L'analyse spectrale permet de conclure qu'il s'agit du composé 12 qui est donc obtenu avec un rendement de 75%.

RMN $^1$H (AC200) ($CDCl_3$)-δ-($C\underline{H}_3$)$_2$CH : centré sur 1,14, 2d en partie superposés (6H), J=6,4 Hz ($C\underline{H}_3$)$_3$C : 1,47, s(9H) ; $C\underline{H}_2NC\underline{H}$, $C\underline{H}_2C\underline{H}O\underline{H}$, $N\underline{H}_2$ ; 3,20-4,20, m (9 H) ; C$\underline{H}$ arom. : 6,61-6,64-6,72 et 6,75, 2d (4H), J=9,0 Hz.

RMN $^{13}$C (AC200) ($CDCl_3$)-δ-($\underline{C}H_3$)$_2$CH : 20,46 et 20,73 ; ($C\underline{H}_3$)$_3$C 28,39 ; ($CH_3$)$_2\underline{C}H$ : 46,85 ; $\underline{C}H_2N$ :48,62 ; $\underline{C}HOH$ : 70,27 ; ($CH_3$)$_3\underline{C}$ : 71,75 ; $O\underline{C}H_2$ : 80,44 ; C$\underline{H}$ arom. : 115,41 et 116,36 ; $\underline{C}NH_2$: 140,11 ; $\underline{C}O$ : 151,68 ; $\underline{C}$=0 : 156,30.

| Analyse | C | H | N |
|---|---|---|---|
| calculée | 62,96 | 8,64 | 8,64 |
| trouvée | 61,95 | 8,69 | 8,39 |

**Exemple 19 : Préparation de l'acide-3-iodoacrylique-(Z) : (composé 13)**

On prépare une solution de 12,5 g (0,208 mol) d'acide propioloique dans 200 ml d'eau. Après avoir porté la température à 75°C, on ajoute en 30 minutes environ 27,5 ml d'acide iodhydrique à 57% (d=1,70) (0,209 mol). On maintient la température pendant 2 heures, puis refroidit et extrait à l'éther (4x50 ml). Après séchage et évaporation du solvant, on obtient 25,6 g de produit brut qui cristallise en partie au bout de quelques heures. Après récupération du solide (8,3 g) le liquide restant est chauffé à 75°C. Sous pression réduite (4-5 mmHg) et après passage d'une fraction de tête à 22°C on arrête le chauffage. Le culot cristallise spontanément par refroidissement (11 g). On obtient donc au toal 19,3 g (47%) de composé
13. P.F = 65-66°C.

RMN $^1$H (AC200) ($CDCl_3$)-δ-=C$\underline{H}$COOH : 6,90 et 6,94, d(1H), J=9,1 Hz ; C$\underline{H}$= : 7,60 et 7,64, d(1H), J=9,1Hz ; COO$\underline{H}$ : 9,84, s(1H).

**Exemple 20 : Préparation du 1-[4-(2-iodoéthylènamido)phénoxy]-3-N-isopropyl-N-tert-butoxycarbonylaminopropan-2-ol racémique : (composé 14)**

A partir de 153 mg ($0{,}47.10^{-3}$ mol) du composé 12 en solution dans 10 ml de DMF anhydre, de 97,5 mg ($0{,}47.10^{-3}$ mol) de DCC, de 63,5 mg ($0{,}47.10^{-3}$ mol) de HOBT et de 93,5 mg ($0{,}47.10^{-3}$ mol) de composé 13. On obtient après purification sur gel de silice (éluant : chloroforme-acétate d'éthyle : 9/1). 115 mg (49%) de composé 14 sous forme d'une huile.

RMN $^1$H (AC200) ($CDCl_3$)-δ-CH($C\underline{H}_3$)$_2$ : 1,12-1,28, 2d (6H), J=6,5 Hz ; C($C\underline{H}_3$)$_3$ : 1,48, s(9H) ; $OC\underline{H}_2CHO\underline{H}C\underline{H}_2NC\underline{H}$ : 3,37-4,31, m (7H) ; N$\underline{H}$ : 4,95, s (1H) ; C$\underline{H}$ arom. : 6,85-6,90, d(2H), J=8,98 Hz ; =C$\underline{H}$I : 6,95-7,00, d(1H), J=8,9 Hz ; C$\underline{H}$=CHI : 7,16-7,21, d(1H), J=8,9 Hz ; C$\underline{H}$ arom. : 7,49-7,53, d(2H) ; N$\underline{H}$ : 7,93, s large.

RMN $^{13}$C (AC200) ($CDCl_3$)-δ-CH($\underline{C}H_3$)$_2$ : 20,49-20,79 ; C($\underline{C}H_3$)$_3$ : 28,42 ; $\underline{C}H_2N$ : 46,74 ; $\underline{C}H(CH_3)_2$ : 48,64 ;

CHOH : 69,91 ; O$\underline{C}H_2$: 71,87 ; $\underline{C}(CH_3)_3$: 80,66 ; =$\underline{C}$HI : 89,22 ; $\underline{C}$H arom. : 114,71 et 121,76 ; $\underline{C}$N : 130,64 ; $\underline{C}$H=CHI : 132,87 ; $\underline{C}$O : 155,65 ; $\underline{C}$OO : 157,52 ; $\underline{C}$O : 162,22.

SM/IC : ($NH_3^+$) isobutane) : 522 ($MH+NH_3$) ; 505 (MH) ; 449 ($MH-C(CH_3)_3$ ; 377 (M-I) ; 323 (M-COCH=CHI).

**Exemple 21 : Préparation du 1-[4-(2-iodoéthylènamido)phénoxy]-3-isopropylaminopropan-2-ol racémique : (composé 15)**

On place sous argon une solution de 30 mg ($0,6.10^{-4}$ mol) de compose 14 dans 1 ml d'acétonitrile anhydre. Après addition de 10µl ($0,7.10^{-4}$ mol) d'iodure de triméthysilane, on laisse agiter à température ambiante. La CCM (éluant : chloroforme-méthanol) montre la disparition du produit de départ au bout de deux heures. On ajoute alors 4 équivalents de méthanol, agite cinq minutes puis évapore les solvants. On obtient 19,9 mg de produit après purification sur colonne sous forme de cristaux blancs. (Rdt : 80%). P.F = dégradation à partir de 170°.

RMN $^1H$ (AC200) ($C_3OD$)-δ-$CH(C\underline{H}_3)_2$ : 1,25-1,34, 2d (6H), J=6,4 Hz ; $C\underline{H}_2NC\underline{H}(CH_3)_2$ : 3,14-3,29, m(3H) ; $OC\underline{H}_2$CHOH: 3,87-4,00, m(2H) ; C$\underline{H}$OH : 4,07-4,23, m(1H) ; C$\underline{H}$ arom. : 6,81-6,94, d(2H), J=8,9 Hz ; CH=C$\underline{H}$I : 6,97-7,06, d(1H), J-9,6Hz ; C$\underline{H}$=CHI : 7,22-7,31, d (1H), J=9,6 Hz ; C$\underline{H}$ arom. et N$\underline{H}$CO : 7,41-7,52, m (3H).

RMN $^{13}C$ (AC200) ($CD_3OD$)-δ-$CH(\underline{C}H_3)_2$ : 18,93-19,23 ; $\underline{C}H_2$N: 46, 52 ; $\underline{C}H(CH_3)_2$ : 48,44 ; $\underline{C}$HOH : 69,85 ; O$\underline{C}H_2$: 71,81 ; CH=$\underline{C}$HI : 88,97 ; $\underline{C}$H arom.: 114,60 et 121,66 ; $\underline{C}$N : 130,23 ; $\underline{C}$H=CHI : 132,31 ; $\underline{C}$O : 155,21 ; $\underline{C}$=O : 161,90.

SM/IC : ($NH_3^+$ isobutane) : 405 (MH) ; 225 (M-COCH=CHI) ; 209 (M-NH-COCH=CHI).

Dans le tableau 10 qui suit, on a donné les coefficients de partage octanol-eau des composés 10, 15 et du composé 16 : (1-{4-[2-(2-iodoéthoxy) éthoxy]phénoxy}-3-isopropyl-aminopropan-2-ol)

Tableau 10

| Composé | Coefficient de partage octanol-eau à pH 7 |
|---|---|
| 10 | 13,2 |
| 15 | 2,91 |
| 16 | 6,7 |
| propranol | 11,3 |

**Exemple 22 : étude de biodistribution chez la souris**

L'étude de la biodistribution des radioligands chez la souris, permet d'apprécier l'intensité relative de la fixation cardiaque de la molécule et son évolution dans le temps.

Après marquage à l'iode 123 et purification par CLHP 20µCi environ de produit sont mis en solution dans 5 ml d'une solution : éthanol 20%-sérum physiologique 80%. Après injection intraveineuse de 25 µl de cette solution dans la queue de souris vigiles (souris swiss femelles de 20 à 25 g), celles-ci (5 à chaque fois) sont sacrifiées à différents temps après l'injection. On mesure pour chaque souris les activités cardiaques, hépatique, rénale et pulmonaire. Les résultats sont exprimés en pourcentage de la dose injectée par gramme d'organe.

Le CGP12177 étant la référence, un lot de souris a été étudié après injection de CGP 12177 marqué au tritium.

**Résultats**

Tableau 11 :

| **Biodistribution du [$^3H$]CGP12177 (référence)** | | | | |
|---|---|---|---|---|
| Temps | 1 min. | 2 min. | 5 min. | 10 min. |
| Coeur | 9 ± 1,4 | 7,2 ± 0,9 | 7,0 ± 1 | 6,7 ± 1,3 |
| Poumon | 66,3 ± 9,2 | 44,3 ± 8,6 | 37,9 ± 9,3 | 42 ± 8,5 |

Les valeurs données (tableau 11) montrent que dans le myocarde, l'activité du $^3H$ CGP 12177 atteint un plateau dès la 2ème minute après l'injection, et que cette activité représente environ 7% de la dose injectée par gramme d'organe.

Tableau 12 :

| Biodistribution du composé 16 | | | | |
|---|---|---|---|---|
| Temps | Coeur | Foie | Poumon | Rein |
| 15" | 20,4 ± 3,8 | 1,5 ± 0,8 | 112 ± 37 | 4 ± 2,6 |
| 2' | 13 ± 1,3 | 3,6 ± 0,5 | 73,3 ± 10 | 16,4 ± 3,6 |
| 5' | 7 ± 0,7 | 7,2 ± 1,6 | 52,7 ± 4 | 19,5 ± 5 |
| 10' | 4,1 ± 0,4 | 7,8 ± 1,6 | 38,9 ± 5,3 | 15,4 ± 2 |
| 15' | 3,6 ± 0,4 | 7,9 ± 0,5 | 29,6 ± 4 | 14 ± 2,6 |

Cette molécule a une fixation cardiaque intéressante puisqu'il reste un pourcentage de radioactivité dans le coeur jusqu'à 15 minutes.

Tableau 13 :

| Biodistribution du composé 10 | | | | |
|---|---|---|---|---|
| Temps | Coeur | Poumon | Foie | Rein |
| 15" | 28,4 ± 3,6 | 144,52 | 1,7 ± 0,8 | 11,4 ± 5,1 |
| 2' | 15 ± 1,5 | 60 ± 10 | 7,2 ± 1,1 | 14,8 ± 1 |
| 5' | 9,5 ± 2,3 | 58 ± 4 | 10,2 ± 1,1 | 14 ± 1,1 |
| 10' | 5,3 ± 0,15 | 33 ± 14 | 6,3 ± 3,3 | 10,7 ± 1,1 |
| 15' | 3,9 ± 0,2 | 2,3 ± 7 | 9 ± 0,2 | 9,1 ± 0,4 |
| 30' | 3,1 ± 0,15 | 22 ± 4 | 7,2 ± 0,8 | 7,6 ± 0,2 |

Ce composé présente une bonne fixation cardiaque : (3,1% après 30 minutes) ; la lipophilie de la molécule est confirmée, puisqu'on trouve un pourcentage de 22% après 30 minutes dans les poumons.

Tableau 14 :

| Biodistribution du composé 15 | | | | |
|---|---|---|---|---|
| Temps | Coeur | Poumon | Foie | Rein |
| 15" | 16,2 ± 2,1 | 55 ± 12 | 4,8 ± 0,2 | 17,1 ± 1,2 |
| 2' | 14,4 ± 0,9 | 34 ± 6 | 6 ± 0,75 | 20,9 ± 2,5 |
| 5' | 8,4 ± 0,3 | 25 ± 6 | 3,6 ± 0,4 | 13,3 ± 0,6 |
| 10' | 3,6 ± 0,7 | 13,2 ± 3,1 | 2,6 ± 0,2 | 7,2 ± 0,4 |
| 15' | 3,2 ± 0,2 | 11,2 ± 2,2 | 2,6 ± 0,2 | 6,3 ± 1 |
| 30' | 2,2 ± 0,15 | 8,4 ± 0,8 | 2,1 ± 0,1 | 4,8 ± 0,6 |

Ainsi, les composés de l'invention, notamment le composé 6,répondent à tous les critères que doit présenter une molécule pour être un marqueur des récepteurs β adrénergiques. En effet, on a montré que le composé possède :

- une lipophilie inférieure à celle du propranolol,
- une affinité vis-à-vis des récepteurs β ($CI_{50}$=2,04.$10^{-6}$) voisine de celle du propranolol ($CI_{50}$=1,93$10^{-7}$),
- un effet antagoniste pur (pourcentage d'inhibition = 116%) vis-à-vis de celui de l'isoprénaline,
- une très bonne fixation cardiaque.

**Revendications**

1. Dérivé halogéné de 1-phénoxy-3-N-alkyl aminopropan-2-ol répondant à la formule :

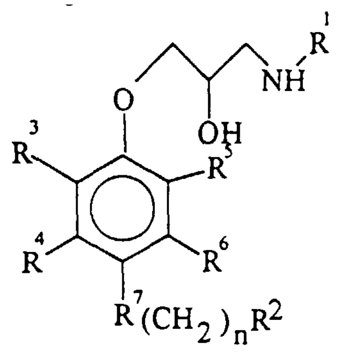

(I)

dans laquelle $R^1$ est un groupe alkyle, un groupe aryle, un groupe aralkyle éventuellement substitué sur sa partie aryle par des groupes alkyle ou alcoxy, ou un groupe $-CH_2-CH_2-Y^1-R^8$ avec $Y^1$ représentant O, S, NHCO, CONH ou NHCONH et $R^8$ représentant un groupe alkyle ou aryle éventuellement substitué,

- $R^2$ représente un groupe répondant à l'une des formules suivantes :
- CH = CHX
- C ≡ CX
- $O-(CH_2)_2X$
- $OCH_2CH = CHX$

avec X représentant un atome d'halogène ou un atome d'un isotope radioactif d'un halogène,

- $R^3$, $R^4$, $R^5$ et $R^6$ qui peuvent être identiques ou différents, représentent un atome d'hydrogène, un atome d'halogène, un groupe hydrocarboné saturé ou insaturé, un groupe aryle, OH, CN, $NH_2$, ou un groupe de formule NHR, $NR_2$, COR, CONHR ou COOR avec R représentant un groupe alkyle ou aryle éventuellement substitué,
- $R^7$ représente O ou NHCO, et
- n est égal à 0,1 ou 2.

**2.** Dérivé selon la revendication 1, dans lequel le carbone qui porte le groupe OH présente la configuration soit R, soit S, soit R et S dans des proportions variables.

**3.** Dérivé selon l'une quelconque des revendications 1 et 2, caractérisé en ce que le dérivé répond à la formule :

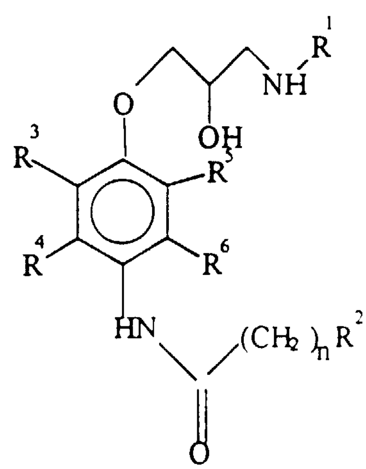

(II)

dans laquelle n, $R^1$, $R^3$, $R^4$, $R^5$ et $R^6$ sont tels que définis dans la revendication 1 et $R^2$ représente CH=CHI.

**4.** Dérivé selon l'une quelconque des revendications 1 et 2, caractérisé en ce qu'il répond à la formule :

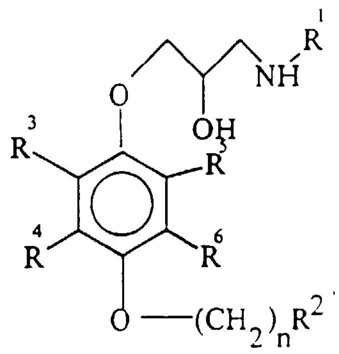

(III)

dans laquelle n, $R^1$, $R^3$, $R^4$, $R^5$ et $R^6$ sont tels que définis dans la revendication 1 et $R^2$ est le groupe O-$(CH_2)_2$-I, $O(CH_2)_2Cl$ ou $OCH_2CH{=}CHI$.

**5.** Dérivé selon l'une quelconque des revendications 1 à 4, caractérisé en ce que $R^1$ est le groupe isopropyle.

**6.** Dérivé selon l'une quelconque des revendications 1 à 5, caractérisé en ce que X représente I ou Cl.

**7.** Dérivé selon l'une quelconque des revendications 1 à 5, caractérisé en ce que X représente $^{123}I$, $^{125}I$ ou $^{131}I$.

**8.** 1-[4-(4-iodobut-3-énamido)phénoxy]-3-N-isopropylaminopropan-2-ol.

**9.** 1-[4-(5-iodo-2-oxapent-4-ényloxy)phénoxy] -3-isopropylaminopropan-2-ol.

**10.** 1-[4-(2-iodoéthylénamido)phénoxy]-3-isopropylaminopropan-2-ol.

**11.** 1-{4-[2-(2-iodoéthoxy)éthoxy]phénoxy}-3-isopropyl-aminopropan-2-ol.

**12.** Dérivé selon l'une quelconque des revendications 8 à 11, caractérisé en ce que l'iode est $^{123}I$, $^{125}I$ ou $^{131}I$.

**13.** Procédé de préparation d'un dérivé halogéné de formule

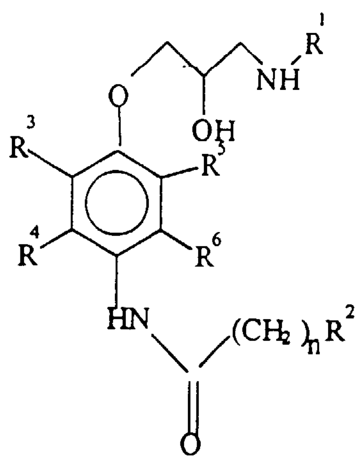

(II)

dans laquelle $R^1$ a la signification donnée dans la revendication 1, $R^2$ représente -CH=CHX ou -C≡CX, avec X étant un atome d'halogène non radioactif, et n est égal à 0,1 ou 2, caractérisé en ce qu'il comprend :

a) la réaction d'un 4-aminophénol avec un acide de formule $R^2$-$(CH_2)_n$-COOH pour obtenir un amide de formule :

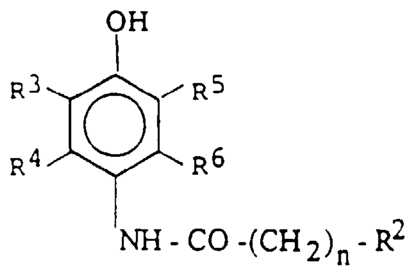

b) la glycidylation de l'amide ainsi obtenu par réaction avec le tosylate de glycidyle pour obtenir un époxyde de formule :

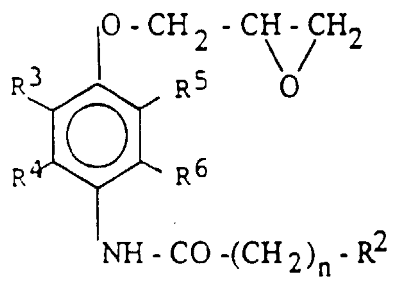

c) la réaction de l'époxyde avec une amine de formule $R^1NH_2$ pour obtenir le dérivé halogéné de formule (II).

**14.** Procédé selon la revendication 13, caractérisé en ce que le tosylate de glycidyle utilisé en b) est soit R, soit S, soit R et S.

**15.** Procédé selon l'une quelconque des revendications 13 et 14, caractérisé en ce qu'il comprend une étape complémentaire d'échange de l'atome d'halogène X par le même halogène X radioactif.

**16.** Procédé selon l'une quelconque des revendications 13 et 14, caractérisé en ce que l'on prépare l'acide de formule $R^2$-$(CH_2)_n$COOH avec $R^2$ représentant CH=CHI par réaction d'un ester méthylique de formule :

$$CH{\equiv}C\text{-}(CH_2)_n\text{-}COOCH_3$$

avec l'hydrure de tributylétain pour former l'ester de formule :

$$(nC_4H_9)_3Sn\text{-}CH{=}CH\text{-}(CH_2)_n\text{-}COOCH_3,$$

suivie d'une halogénation par un agent halogénant approprié et d'une hydrolyse de l'ester halogéné obtenu.

**17.** Composition pharmaceutique, caractérisée en ce qu'elle comprend un dérivé halogéné de 1-phénoxy-3-N-alkylamino-propan-2-ol selon l'une quelconque des revendications 1 à 6 et 8 à 11.

**18.** Composition pharmaceutique selon la revendication 17, dans laquelle le dérivé halogéné est un β-bloquant.

**19.** Composition radiopharmaceutique, caractérisée en ce qu'elle comprend un dérivé halogéné de l-phénoxy-3-N-alkylamino-propan-2-ol selon l'une quelconque des revendications 1 à 5, 7 et 12 dans lequel l'atome d'halogène est un atome d'halogène radioactif.

**20.** Composition selon la revendication 19 destinée à l'imagerie médicale.

**21.** Composition selon la revendication 19 destinée à la visualisation des récepteurs β -adrénergiques.

## Patentansprüche

1. Halogenderivat von 1-Phenoxy-3-N-alkyl-aminopropan-2-ol der Formel

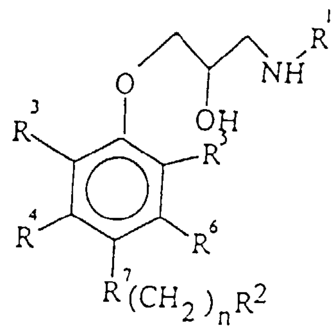

(I)

worin bedeuten:

| | |
|---|---|
| $R^1$ | eine Alkylgruppe, eine Arylgruppe, eine Aralkylgruppe, die an ihrem Aryl-Teil gegebenenfalls durch Alkyl- oder Alkoxygruppen substituiert ist, oder eine Gruppe $-CH_2-CH_2-Y^1-R^8$, worin $Y^1$ für O, S, NHCO, CONH oder NHCONH und $R^8$ für eine gegebenenfalls substituierte Alkyl- oder Arylgruppe stehen, |
| $R^2$ | eine Gruppe mit einer der folgenden Formeln<br>- $CH = CHX$<br>- $C \equiv CX$<br>- $O-(CH_2)_2X$<br>- $OCH_2CH = CHX$<br>worin X für ein Halogenatom oder ein radioaktives Isotop eines Halogens steht, |
| $R^3$, $R^4$, $R^5$ und $R^6$, | die gleich oder verschieden sein können, ein Wasserstoffatom, ein Halogenatom, eine gesättigte oder ungesättigte Kohlenwasserstoffgruppe, eine Arylgruppe, OH, CN, $NH_2$ oder eine Gruppe der Formel NHR, $NR_2$, COR, CONHR oder COOR, worin R für eine gegebenenfalls substituierte Alkyl- oder Arylgruppe steht, |
| $R^7$ | O oder NHCO und |
| n | die Zahl 0, 1 oder 2. |

2. Derivat nach Anspruch 1, in dem das Kohlenstoffatom, das die OH-Gruppe trägt, in der Konfiguration R oder S oder R,S in variablen Mengenan-teilen vorliegt.

3. Derivate nach einem der Ansprüche 1 und 2, dadurch gekennzeichnet, daß es der Formel entspricht

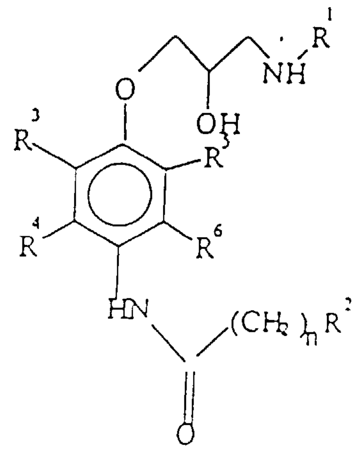

(II)

in der n, $R^1$, $R^3$, $R^4$, $R^5$ und $R^6$ wie in Anspruch 1 definiert sind und $R^2$ für CH=CHI steht.

**4.** Derivat nach einem der Ansprüche 1 und 2, dadurch gekennzeichnet, daß es der Formel entspricht

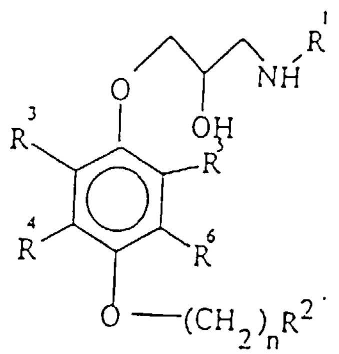

(III)

in der n, $R^1$, $R^3$, $R^4$, $R^5$ und $R^6$ wie in Anspruch 1 definiert sind und $R^2$ für die Gruppe $O\text{-}(CH_2)_2\text{-}I$, $O\text{-}(CH_2)_2C\ell$ oder $OCH_2CH{=}CHI$ steht.

**5.** Derivat nach einem der Ansprüche 1 bis 4, dadurch gekennzeichnet, daß $R^1$ für die Isopropyl-Gruppe steht.

**6.** Derivat nach einem der Ansprüche 1 bis 5, dadurch gekennzeichnet, daß X für I oder $C\ell$ steht.

**7.** Derivat nach einem der Ansprüche 1 bis 5, dadurch gekennzeichnet, daß X für $^{123}I$, $^{125}I$ oder $^{131}I$ steht.

**8.** 1-[4-(4-Iodobut-3-enamido)phenoxy]-3-N-isopropylaminopropan-2-ol.

**9.** 1-[4-(5-Iodo-2-oxapent-4-enyloxy)phenoxy]-3-isopropylaminopropan-2-ol.

**10.** 1-[4-(2-Iodoethylenamido)phenoxy]-3-isopropylaminopropan-2-ol.

**11.** 1-{4-(2-(2-Iodoethoxy)ethoxy]phenoxy}-3-isopropyl-aminopropan-2-ol.

**12.** Derivat nach einem der Ansprüche 8 bis 11, dadurch gekennzeichnet, daß es sich bei dem Iod um $^{123}I$, $^{125}I$ oder $^{131}I$ handelt.

**13.** Verfahren zur Herstellung eines Halogenderivats der Formel

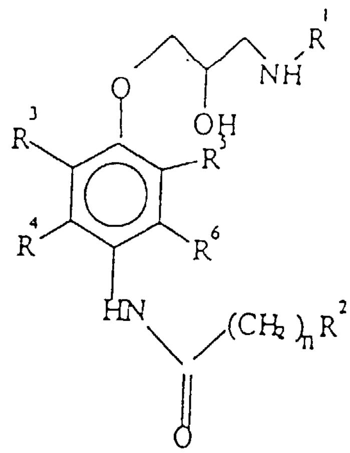

(II)

worin $R^1$ die in Anspruch 1 angegebene Bedeutung hat, $R^2$ für -CH=CHX oder -C≡CX steht, wobei X ein nicht radioaktives Halogenatom bedeutet, und n für die Zahl 0, 1 oder 2 steht, dadurch gekennzeichnet, daß es umfaßt:

a) die Umsetzung eines 4-Aminophenols mit einer Säure der Formel $R^2-(CH_2)_n-COOH$ zur Bildung eines Amids der Formel

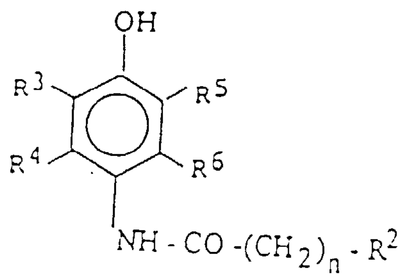

b) die Glycidylierung des auf diese Weise erhaltenen Amids durch Umsetzung mit dem Glycidyltosylat zur Bildung eines Epoxids der Formel

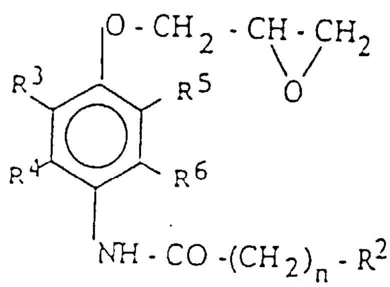

c) die Umsetzung des Epoxids mit einem Amin der Formel $R^1NH_2$ zur Bildung des Halogenderivats der Formel (II).

**14.** Verfahren nach Anspruch 13, dadurch gekennzeichnet, daß das in der Stufe (b) verwendete Glycidyltosylat in der R- oder S- oder R,S-Konfiguration vorliegt.

**15.** Verfahren nach einem der Ansprüche 13 und 14, dadurch gekennzeichnet, daß es eine zusätzliche Stufe zum Austausch des Halogenatoms X gegen das gleiche radioaktive Halogenatom X umfaßt.

**16.** Verfahren nach einem der Ansprüche 13 und 14, dadurch gekennzeichnet, daß man die Säure der Formel $R^2$-$(CH_2)_n$-COOH, worin $R^2$ für CH=CHI steht, herstellt durch Umsetzung eines Methylesters der Formel

$$CH{\equiv}C\text{-}(CH_2)_n\text{-}COOCH_3$$

mit Tributylzinnhydrid zur Bildung des Esters der Formel

$$(nC_4H_9)_3Sn\text{-}CH{=}CH\text{-}(CH_2)_n\text{-}COOCH_3,$$

woran sich eine Halogenierung mit einem geeigneten Halogenierungsmittel und eine Hydrolyse des erhaltenen halogenierten Esters anschließen.

**17.** Pharmazeutische Zusammensetzung, dadurch gekennzeichnet, daß sie ein Halogenderivat von 1-Phenoxy-3-N-alkyl-aminopropan-2-ol nach einem der Ansprüche 1 bis 6 und 8 bis 11 enthält.

**18.** Pharmazeutische Zusammensetzung nach Anspruch 17, in der das Halogenderivat ein β-Blocker ist.

**19.** Radiopharmazeutische Zusammensetzung, dadurch gekennzeichnet, daß sie ein Halogenderivat von 1-Phenoxy-3-N-alkyl-aminopropan-2-ol nach einem der Ansprüche 1 bis 5, 7 und 12 enthält, in dem das Halogenatom ein radioaktives Halogenatom ist.

**20.** Zusammensetzung nach Anspruch 19 für die bildliche Darstellung in der Medizin.

**21.** Zusammensetzung nach Anspruch 19 für die Sichtbarmachung von β-adrenergischen Rezeptoren.

**Claims**

**1.** Halogenated 1-phenoxy-3-N-alkylamino-2-propanol derivative corresponding to the formula:

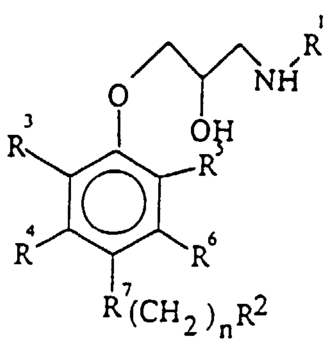

(I)

in which $R^1$ is an alkyl group, an aryl group, an aralkyl group optionally substituted on its aryl part with alkyl or alkoxy groups, or a group $-CH_2-CH_2-Y^1-R^8$ with $Y^1$ representing O, S, NHCO, CONH or NHCONH and $R^8$ representing an optionally substituted alkyl or aryl group,

- $R^2$ represents a group corresponding to one of the following formulae:
- CH=CHX
- C≡CX
- O-$(CH_2)_2$X
- $OCH_2CH$=CHX

with X representing a halogen atom or an atom of a radioactive isotope of a halogen,

- $R^3$, $R^4$, $R^5$ and $R^6$, which may be identical or different, represent a hydrogen atom, a halogen atom, a saturated

or unsaturated hydrocarbon-based group, an aryl, OH, CN or $NH_2$ group or a group of formula NHR, $NR_2$, COR, CONHR or COOR with R representing an optionally substituted alkyl or aryl group,

- $R^7$ represents O or NHCO, and
- n is equal to 0, 1 or 2.

**2.** Derivative according to Claim 1, in which the carbon which bears the OH group has either R or S or R and S configuration in variable proportions.

**3.** Derivative according to either of Claims 1 and 2, characterized in that the derivative corresponds to the formula:

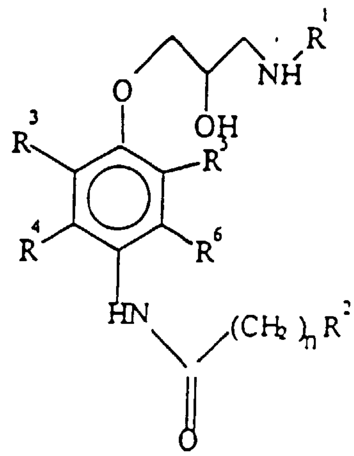

(II)

in which n, $R^1$, $R^3$, $R^4$, $R^5$ and $R^6$ are as defined in Claim 1 and $R^2$ represents CH=CHI.

**4.** Derivative according to either of Claims 1 and 2, characterized in that it corresponds to the formula:

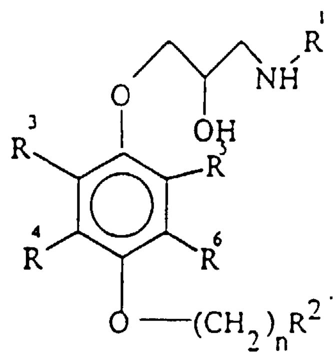

(III)

in which n, $R^1$, $R^3$, $R^4$, $R^5$ and $R^6$ are as defined in Claim 1 and $R^2$ is the group O-$(CH_2)_2$-I, $O(CH_2)_2Cl$ or $OCH_2CH$=CHI.

**5.** Derivative according to any one of Claims 1 to 4, characterized in that $R^1$ is an isopropyl group.

**6.** Derivative according to any one of Claims 1 to 5, characterized in that X represents I or Cl.

**7.** Derivative according to any one of Claims 1 to 5, characterized in that X represents $^{123}I$, $^{125}I$ or $^{131}I$.

**8.** 1-[4-(4-Iodobut-3-enamido)phenoxy]-3-N-isopropylamino-2-propanol.

**9.** 1-[4-(5-Iodo-2-oxapent-4-enyloxy)phenoxy]-3-isopropylamino-2-propanol.

**10.** 1-[4-(2-Iodoethyleneamido)phenoxy]-3-isopropylamino-2-propanol.

**11.** 1-{4-[2-(2-Iodoethoxy)ethoxy]phenoxy}-3-isopropylamino-2-propanol.

**12.** Derivative according to any one of Claims 8 to 11, characterized in that the iodine is $^{123}I$, $^{125}I$ or $^{131}I$.

**13.** Process for the preparation of a halogenated derivative of formula

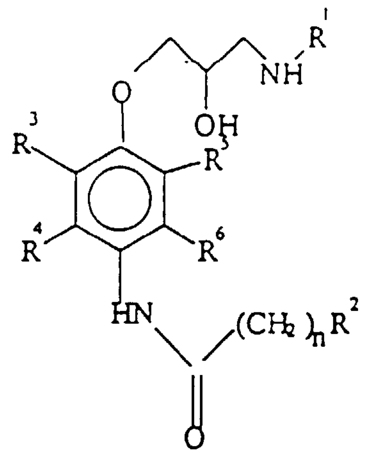

(II)

in which $R^1$ has the meaning given in Claim 1, $R^2$ represents -CH=CHX or -C≡CX, with X being a nonradioactive halogen atom, and n is equal to 0, 1 or 2, characterized in that it comprises:

a) the reaction of a 4-aminophenol with an acid of formula $R^2$-$(CH_2)_n$-COOH in order to obtain an amide of formula:

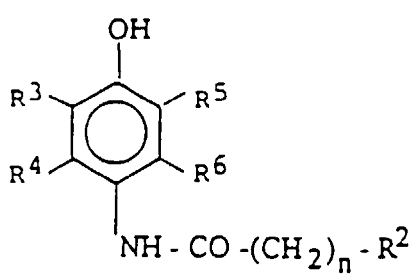

b) glycidylation of the amide thus obtained by reaction with glycidyl tosylate in order to obtain an epoxide of formula:

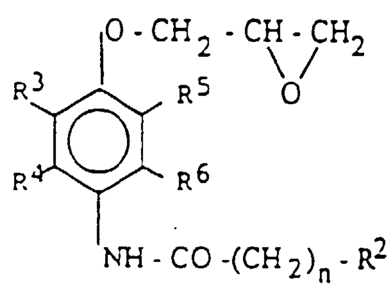

c) reaction of the epoxide with an amine of formula $R^1NH_2$ in order to obtain the halogenated derivative of formula (II).

**14.** Process according to Claim 13, characterized in that the glycidyl tosylate used in b) is either R or S or R and S.

**15.** Process according to either of Claims 13 and 14, characterized in that it comprises an additional step of exchanging the halogen atom X with the same radioactive halogen X.

**16.** Process according to either of Claims 13 and 14, characterized in that the acid of formula $R^2$-$(CH_2)_nCOOH$, with $R^2$ representing CH=CHI, is prepared by reaction of a methyl ester of formula:

$$CH{\equiv}C\text{-}(CH_2)_n\text{-}COOCH_3$$

with tributyltin hydride in order to form the ester of formula:

$$(nC_4H_9)_3Sn\text{-}CH{=}CH\text{-}(CH_2)_n\text{-}COOCH_3$$

followed by halogenation with a suitable halogenating agent and hydrolysis of the halogenated ester obtained.

**17.** Pharmaceutical composition, characterized in that it comprises a halogenated 1-phenoxy-3-N-alkylamino-2-propanol derivative according to any one of Claims 1 to 6 and 8 to 11.

**18.** Pharmaceutical composition according to Claim 17, in which the halogenated derivative is a β-blocker.

**19.** Radiopharmaceutical composition, characterized in that it comprises a halogenated 1-phenoxy-3-N-alkylamino-2-propanol derivative according to any one of Claims 1 to 5, 7 and 12, in which the halogen atom is a radioactive halogen atom.

**20.** Composition according to Claim 19, intended for medical imaging.

**21.** Composition according to Claim 19, intended for visualizing β-adrenergic receptors.